# EUROPÄISCHE PATENTSCHRIFT

(11) **EP 4 057 951 B1**
(45) Veröffentlichungstag und Bekanntmachung des Hinweises auf die Patenterteilung: **25.03.2026**
(21) Anmeldenummer: 20808090.3
(22) Anmeldetag: 16.11.2020
(51) Int. Cl.: A61F 5/01, F16F 9/36, F16F 9/504

(54) **VORRICHTUNG ZUR STABILISIERUNG VON BEWEGUNGEN ZWEIER SICH RELATIV ZUEINANDER BEWEGBARER TEILE EINES KÖRPERBEREICHS UND/ODER EINES SPORTGERÄTS UMFASSEND EINE WIRKKÖRPERANORDNUNG MIT DICHTLIPPE**
APPARATUS FOR STABILISING MOVEMENTS OF TWO PARTS OF A BODY REGION AND/OR OF A SPORTS DEVICE WHICH ARE MOVABLE RELATIVE TO ONE ANOTHER, COMPRISING AN ACTIVE BODY ARRANGEMENT HAVING A SEALING LIP
APPAREIL DE STABILISATION DE MOUVEMENTS DE DEUX PARTIES D'UNE RÉGION DE CORPS ET/OU D'UN DISPOSITIF DE SPORT MOBILES L'UNE PAR RAPPORT À L'AUTRE, COMPRENANT UN AGENCEMENT DE CORPS ACTIF DOTÉ D'UNE LÈVRE D'ÉTANCHÉITÉ

(30) Priorität: 15.11.2019 DE 102019130999
(43) Veröffentlichungstag der Anmeldung: 21.09.2022
(73) Patentinhaber: Betterguards Technology GmbH, 13585 Berlin (DE)
(72) Erfinder: BICHLER, Vinzenz, 10777 Berlin (DE); STUMPER, Timo, 10963 Berlin (DE); BUSCHINGER, Oscar, 10707 Berlin (DE)
(74) Vertreter: Okoampah, Rene
(86) Internationale Anmeldenummer: PCT/EP2020/082268
(87) Internationale Veröffentlichungsnummer: WO 2021/094611

(56) Entgegenhaltungen:
- EP-A1- 2 842 527
- EP-A1- 3 092 980
- EP-B1- 1 437 524
- US-A- 5 190 126
- US-A- 5 730 263
- US-A1- 2014 015 176
- US-A1- 2016 213 549
- US-A1- 2017 304 057

## Beschreibung

### Technisches Gebiet

Die vorliegende Erfindung betrifft eine Vorrichtung zur Stabilisierung von Bewegungen zweier sich relativ zueinander bewegbarer Teile eines Körperbereichs und/oder eines Sportgeräts.

### Stand der Technik

Es ist bekannt Körpergelenke, Muskeln und Sehnen mittels Vorrichtungen, welche eine adaptive Bewegungsbegrenzung ermöglichen zu stabilisieren. Ferner ist es bekannt Sportgeräte die ruckartigen Bewegungen ausgesetzt werden können, mit adaptiven Bewegungsbegrenzungsvorrichtungen zu versehen.

Unter anderem wird das adaptive Verhalten solcher Vorrichtungen dadurch erreicht, dass sich zwei Körper relativ zueinander bewegen, wobei sich zwischen den Körpern ein Füllmedium befindet. Dabei kann ein Körper der Vorrichtung eine Aufnahme bilden, die mit dem Füllmedium gefüllt ist. Der andere Körper kann einen Auszugskörper bilden, welcher in der Aufnahme bewegbar angeordnet ist. In dem Bereich zwischen der Aufnahme und dem Auszugskörper kann das Füllmedium strömen, wenn sich die beiden Körper relativ zueinander bewegen. Die Fließgeschwindigkeit des Füllmediums hängt entscheidend von der Querschnittfläche senkrecht zu einer relativen Verschieberichtung der Aufnahme und des Auszugskörpers ab. Diese zur Strömung für das Füllmedium bereitstehende Querschnittfläche wird auch als hydraulischer Durchmesser bezeichnet und ist letztendlich für das reaktive Verhalten der Vorrichtung bei äußerer Krafteinwirkung entscheidend.

So kann durch die Wahl des hydraulischen Durchmessers der Widerstand festgelegt werden, den die Vorrichtung äußeren Kräften entgegenbringt. Die Vorrichtungen können zwischen zwei Körperstellen eines Anwenders oder zwei sich relativ zueinander bewegbaren Elementen eines Sportgeräts fixiert werden.

Werden über die beiden Körperstellen des Anwenders physiologische Kräfte oder physiologische Geschwindigkeiten, das heißt für das entsprechend zu stabilisierende Körperteil oder Bauteil unkritische Kräfte, in die Vorrichtung eingeleitet, so wird gemäß dem hydraulischen Durchmesser in der Vorrichtung eine entsprechende Relativbewegung der Aufnahme und des Auszugskörpers und damit eine Bewegung des zu stabilisierenden Körperteils zugelassen.

Werden hingegen unphysiologische Kräfte, das heißt für das entsprechend zu stabilisierende Körperteil oder Bauteil kritische Kräfte, in die Vorrichtung eingeleitet, ist aufgrund der Veränderung des hydraulischen Durchmessers eine Relativbewegung zwischen dem Auszugskörper und der Aufnahme nur noch unter sehr hohem Kraftaufwand möglich. Die Vorrichtung blockiert.

Eine solche Vorrichtung ist beispielsweise aus dem US Patent 5, 712, 011 bekannt. US 5, 712, 011 zeigt eine Vorrichtung mit zwei relativ zueinander bewegbaren Körpern. Ein erster Körper umfasst eine Aufnahme, die mit einem Fluid gefüllt ist. Der zweite Körper erstreckt sich zumindest teilweise in die Aufnahme des ersten Körpers und ist dazu konfiguriert, mit dem Fluid eine Wechselwirkung einzugehen. Durch die Wechselwirkung können Relativbewegungen zwischen dem ersten und dem zweiten Körper gedämpft werden.

In der Vorrichtung können im blockierten Zustand sehr hohe Druckbelastungen entstehen, die die Vorrichtung beschädigen können. Eine zu harte bzw. spröde Materialauswahl der Aufnahme hat den Nachteil, dass die Aufnahme zwar den Druckbelastungen standhält, jedoch den Tragekomfort der Vorrichtungen an Körperteilen, -gelenken beeinträchtigt. Auf der anderen Seite hat eine zu elastische Materialauswahl der Aufnahme zur Folge, dass sich die Aufnahme bei hohen Drücken nach außen wölbt, sozusagen ausbaucht und das Füllmedium aus einem im blockierten Zustand komprimierten Raum entlang der Innenwand der Aufnahme vorbei an der Dichtung bzw. an dem Wirkkörper und einem daran angebrachten Dichtring entweichen kann. Dadurch blockiert die Vorrichtung nicht mehr wie gewünscht und das Wirkprinzip der Vorrichtung wird beeinträchtigt.

Es hat sich gezeigt, dass sich O-Ringe an die veränderten Durchmesser der Aufnahme, welche auf die Wölbung der Aufnahme bei einem entsprechenden Druck zurückzuführen sind, nicht anpassen können. Des Weiteren hat sich gezeigt, dass O-Ringe aufgrund der hohen Druckbelastungen ihre Position in der Aufnahme nicht immer halten können. Durch das Entweichen des Füllmediums an der Dichtung vorbei verschiebt sich der O-Ring oder rutscht sogar über den Wirkkörper durch und beeinträchtigt die Funktionsfähigkeit der Vorrichtung bis hin zum Totalausfall, d. h. dass die Vorrichtung zu früh oder zu spät oder gar nicht mehr schaltet, d. h keine Reproduzierbarkeit des Wirkprinzips der Vorrichtung mehr zulässt.

Des Weiteren weisen die im Stand der Technik bekannten Vorrichtungen den Nachteil auf, dass diese im Bereich der physiologischen Geschwindigkeiten nicht blockieren und die Gefahr besteht, dass ein Wirkkörper bei einer andauernden Bewegung bei solchen Geschwindigkeiten auf die Aufnahme anschlägt, wodurch die Vorrichtung beschädigt werden kann.

Die US 5 730 263 A zeigt einen Vibrationsdämpfer. Die EP 1 437 524 B1 zeigt eine Vorrichtung zur Dämpfung bzw. Abbremsung von beweglichen Möbelteilen von Möbelstücken. Die EP 3 092 980 A1 zeigt eine Vorrichtung zur Stabilisierung von Körpergelenken, Muskeln und Sehnen. Die US 5 190 126 A zeigt einen Stoßdämpfer mit lufthohlraumkontrollierten Öffnungen gemäß dem Oberbegriff des Anspruchs 1. Die US 2016/0213549 A1 zeigt eine tragbare Haltungsunterstützungsvorrichtung.

### Darstellung der Erfindung

Ausgehend von dem bekannten Stand der Technik ist es eine Aufgabe der vorliegenden Erfindung, eine verbesserte Vorrichtung von Bewegungen zweier sich relativ zueinander bewegbarer Teile eines Körperbereichs und/oder eines Sportgeräts anzugeben.

Diese Aufgabe wird mittels einer Vorrichtung mit den Merkmalen des Anspruchs 1 gelöst. Vorteilhafte Ausgestaltungen ergeben sich aus den Unteransprüchen.

Entsprechend wird eine Vorrichtung zur Stabilisierung von Bewegungen zweier sich relativ zueinander bewegbarer Teile eines Körperbereichs, insbesondere zweier sich relativ zueinander bewegbarer Körpergelenke, und/oder eines Sportgeräts angegeben, umfassend eine an einem ersten Teil eines Körperbereichs und/oder eines Sportgeräts fixierbare Aufnahme, wobei die Aufnahme mit einem Füllmedium gefüllt ist, und mindestens eine Wirkkörperanordnung, der in der Aufnahme verschiebbar aufgenommen ist und mit dem Füllmedium interagieren kann, einen an einem zweiten Teil desselben Körperbereichs und/oder desselben Sportgeräts fixierbaren Kraftübertragungskörper zum Übertragen einer äußeren Kraft oder Geschwindigkeit auf den Wirkkörper, wobei der Wirkkörper mindestens eine Durchlassöffnung umfasst, durch welche Füllmedium strömen kann. Die Wirkkörperanordnung weist eine Dichtlippe zum Abdichten eines Spalts zwischen einer Innenseite der Aufnahme und einem seitlichen Bereich einer Außenseite der Wirkkörperanordnung auf, wobei die Dichtlippe an einer Außenseite des Wirkkörpers angeordnet ist. Erfindungsgemäß umfasst der Wirkkörper einen ersten Wirkkörper und einen zweiten Wirkkörper, wobei der erste Wirkkörper und der zweite Wirkkörper in der Aufnahme verschiebbar angeordnet sind und mit dem Füllmedium zu interagieren, wobei der Kraftübertragungskörper die äußere Kraft auf den ersten Wirkkörper überträgt, wobei der zweite Wirkkörper über ein Kopplungselement elastisch mit dem ersten Wirkkörper gekoppelt ist, wobei der zweite Wirkkörper und/oder der erste Wirkkörper mindestens eine Durchlassöffnung aufweist, durch welche das Füllmedium strömen kann, wobei der erste Wirkkörper einen Ventilkörper und der zweite Wirkkörper einen Ventilsitz bilden, wobei die Dichtlippe an dem zweiten Wirkkörper angeordnet ist.

Dadurch kann ein Fluss des Mediums durch die Durchlassöffnung in Abhängigkeit von der Ventilstellung zugelassen oder unterbunden werden.

Beispielsweise ist die Dichtlippe an einer Außenseite des Wirkkörpers in Umfangsrichtung und/oder Oberseite und/ oder Unterseite des Wirkkörpers angeordnet.

Im Inneren der Aufnahme der Vorrichtung können durch die kritischen Verschiebegeschwindigkeiten und der daraus resultierenden Kräfte im blockierten Zustand der Vorrichtung, auf den Wirkkörper hohe Druckbelastungen entstehen. Je nach Durchmesser der Aufnahme können unterschiedlich hohe Druckbelastungen entstehen. Es gilt, desto kleiner der Durchmesser, desto höher sind die maximal auftretenden Druckbelastungen. Beispielsweise können Druckbelastungen von 160 bis 280 bar entstehen.

Solche Druckbelastungen können durch eine entsprechende Auslegung der Aufnahme, d. h. Verstärkung der Wandstärke beherrscht werden. Dies ist jedoch nicht wünschenswert, da dies wiederum Einbußen im Tragekomfort der Vorrichtung an den Körpergelenken oder -bereichen aufgrund eines solchen umfangreichen Aufbaus mit sich bringt, der den Raumbedarf der Vorrichtung in Sportgeräten unnötigerweise vergrößert.

Des Weiteren ist zu beachten, dass eine Druckbelastung umso schwieriger zu beherrschen ist desto kleiner der Durchmesser der Aufnahme ist, was wiederum bedeutetet, dass die Wandstärke noch weiter verstärkt werden müsste. Daher ist die Verstärkung der Wandstärke keine geeignete Lösung für den anwendungsspezifischen Zweck der Vorrichtung, da diese für den Einsatz in Sportgeräten oder an Körperreichen möglichst klein sein soll. Der Begriff "Sportgeräte" umfasst vorliegend auch Sportbekleidung wie zum Beispiel Sport-Büstenhalter, Socken, Schuhe, Handschuhe.

Um den Tragekomfort nicht zu beeinträchtigen bzw. die Wandstärke nicht weiter zu erhöhen ist die Aufnahme derart zu konfigurieren, dass diese nicht zu hart/ spröde (z. B. ein Elastizitätsmodul im Bereich von 1500-1800 MPa, Prüfmethode DIN EN ISO 6721-1 (2018-03)) ist. Besonders bevorzugt ist die Aufnahme aus einem Kunststoff, der hinreichende elastische Eigenschaften besitzt.

Es hat sich gezeigt, dass eine Materialauswahl mit elastischen Eigenschaften für die Aufnahme besonders geeignet ist. Unter Aussetzung von hohen Druckbelastungen wölben sich die Wände der Aufnahme nach außen, sogenanntes Ausbauchen. Die Aufnahme wird innerhalb der zulässigen Spannung leicht verformt. Ein Platzen der Aufnahme wird durch die elastischen Eigenschaften der Aufnahme vermieden. Auch ist eine solche Ausgestaltung der Aufnahme vorteilhaft, weil die elastischen Eigenschaften der Aufnahme die Gelenkfehlbewegung im blockierten Zustand der Vorrichtung nicht abrupt abstoppt, sondern moderat abfedert. Durch diesen abfedernden Effekt kann es zu einer Energieaufnahme bzw. Absorption kommen, wodurch die umliegenden Körperstrukturen / Bauteile weniger belastet werden.

Durch das Ausbauchen wird jedoch der Querschnitt bzw. der Durchmesser der Aufnahme partiell vergrößert, was mangels adäquater Dichtung eine Durchströmung des Spalts mit Füllmedium zur Folge hat und zu einem Druckverlust im Inneren der Aufnahme führen kann. Durch den Infolge des Ausbauches auftretenden Druckverlust wird eine deutlich niedrigere Gegenkraft von dem Wirkkörper erzeugt, welche dazu führt, dass die Vorrichtung nicht mehr blockiert und keine ausreichende Schutzwirkung der Vorrichtung bereitgestellt bzw. aufrechterhalten werden kann. Gegebenenfalls kann der Wirkkörper sogar durchrutschen und auf das obere Ende der Aufnahme aufschlagen, was zu Beschädigungen von Wirkkörper und Aufnahme führen kann.

Durch die Dichtlippe kann dieses Problem jedoch gelöst werden. Die Dichtlippe ermöglicht es, die Aufnahme der Vorrichtung derart elastisch (z. B. ein Elastizitätsmodul im Bereich von 300-700 MPa, Prüfmethode DIN EN ISO 6721-1 (2018-03)) zu gestalten, dass unter hohen Druckbelastungen die Aufnahme zwar ausbaucht, jedoch das Füllmedium nicht bzw. nur zu geringem Teil durch den Spalt entweichen kann, da die Dichtlippe sich an die Veränderungen des Durchmessers der Aufnahme angleicht.

Die Aufnahme kann aus Metall hergestellt sein. Alternativ kann die Aufnahme aus Kunststoff hergestellt sein.

Eine Ausgestaltung der Aufnahme aus Kunststoff ermöglicht die Aufnahme mit elastischen Eigenschaften zu versehen. Dies kann beispielsweise mittels Spritzgussverfahrens erfolgen. In einem Beispiel kann die Aufnahme mit anderen Teilen, z. B. einem Rückstellelement, insbesondere einem Dichtschlauch, welcher für das Rückstellen des Wirkkörpers in den Ausgangszustand zuständig ist, aus einem Teil hergestellt werden. Der Vorteil hierbei ist, dass die Fertigungskosten um ein Vielfaches reduziert werden können, da weniger Teile zusammengefügt werden müssen.

Ein weiterer Vorteil der Dichtlippe besteht darin, dass Toleranzen im Spaltmaß, die durch Entformungsschrägen beim Spritzgießverfahren auftreten können, durch die Dichtlippe ausgeglichen werden. Toleranzen bzw. Varianzen im Spaltmaß führen ebenfalls dazu, dass die Blockierwirkung der Vorrichtung nicht immer in konstanten Kraft- bzw. Geschwindigkeitsbereichen auftreten. Die angleichende Funktion der Dichtlippe an die Spalttoleranzen/ -varianzen gewährleistet somit eine bessere Reproduzierbarkeit des Wirkprinzips der Vorrichtung. Die Dichtung passt sich somit immer einem veränderten Durchmesser der Aufnahme an und sorgt für gleichmäßiges Schalten der Vorrichtung, d. h. gleichmäßiges Blockieren der Vorrichtung bei Einleiten von Zugkräften auf dem Kraftübertragungskörper über einer vorbestimmten Zugkraft bzw. Geschwindigkeit.

Die umlaufende Dichtlippe bietet somit den Vorteil, dass sie einen variierenden Spalt oder Abstand zwischen dem Wirkkörper und einer Innenwand der Aufnahme ausgleicht, also dass sie sich bei einer Auswölbung oder Ausbauchung der Aufnahme mit verformt und somit den Druck in der Aufnahme halten kann, wenn die Vorrichtung blockiert.

Durch die Elastizität der Dichtlippe kann auch ein Eindrücken der Aufnahme des Durchmessers zu einem leicht ovalen Querschnitt ausgeglichen werden. Durch das Eindrücken wird die Aufnahme oval. Die Dichtlippe ist derart elastisch, dass unterschiedliche Spaltgrößen entlang des Umfangs ausgeglichen werden können. Die Dichtlippe wird somit an gewissen Stellen entlang des Umfangs zusammengedrückt und an anderen Stellen ausgedehnt, um Spaltvergrößerung zu kompensieren. Durch ein solches adaptives Verhalten der Dichtlippe kann die Aufnahme elastischer ausgestaltet werden. Dadurch kann der Tragekomfort der Vorrichtung verbessert werden.

Des Weiteren ist die Dichtlippe in einer Aufnahme mit ovalem Querschnitt einsetzbar. Die Dichtlippe ist derart elastisch, dass unterschiedliche Spaltgrößen entlang des Umfangs des Wirkkörpers ausgeglichen werden können. Die Dichtlippe ist somit an gewissen Stellen entlang des Umfangs zusammendrückbar und an anderen Stellen ausdehnbar, um unterschiedliche Spaltgrößen aufgrund des ovalen Querschnitts zu kompensieren.

Bei dem Füllmedium kann es sich um ein newtonsches Fluid, wie zum Beispiel Öl, insbesondere Silikonöl handeln. Alternativ kann auch ein dilatantes Fluid verwendet werden.

Der Wirkkörper und das Füllmedium stehen derart miteinander in Wirkverbindung, dass bei einer auf den Wirkkörper wirkenden Kraft oder Geschwindigkeit unterhalb eines vorbestimmten Schwellwerts der Wirkkörper das Füllmedium durch die Durchlassöffnung verdrängen kann, d. h. das Füllmedium kann relativ zum Wirkkörper innerhalb der Aufnahme strömen. Bei einer auf den Wirkkörper wirkenden Kraft bzw. Geschwindigkeit größer gleich des vorbestimmten Schwellwerts wirkt der Wirkkörper derart auf das Füllmedium, dass ein Strömen des Füllmediums relativ zum Wirkkörper durch die Durchlassöffnung nicht oder nur teilweise möglich ist.

Die Dichtlippe kann auch als Dichtmembran bezeichnet werden.

Erfindungsgemäß weist die Wirkkörperanordnung einen an der Innenseite der Aufnahme anliegenden Bereich der Dichtlippe auf, der in einem Winkel (α, β) zu einem seitlichen Bereich der Außenseite des Wirkkörpers verläuft, wobei der Winkel (α, β) veränderlich ist, um Größenschwankungen des Spalts zwischen der Innenseite der Aufnahme und dem seitlichen Bereich der Außenseite der Wirkkörperanordnung ausgleichen zu können und den Spalt auch bei derartigen Größenschwankungen abzudichten.

Größenschwankung ist vorliegend als eine Veränderung des Abstands zwischen der Innenseite der Aufnahme und dem seitlichen Bereich der Außenseite des Wirkkörpers zu verstehen.

Der Abstand ändert sich insbesondere dadurch, dass sich der innere Durchmesser der Aufnahme abschnittsweise durch das Ausbauchen ändert.

Gemäß einer Ausführungsform kann der veränderliche Winkel (α, β) eine Größenschwankung des Spalts von bis zu 15%, bezogen auf die ursprüngliche Größe des Spalts ausgleichen.

Eine solche adaptive Dichtlippe hat den Vorteil, dass die Aufnahme elastisch ausgestaltet werden kann ohne dabei die Funktionsfähigkeit der Vorrichtung zu beinträchtigen. Des Weiteren kann die Aufnahme dünnwandig ausgestaltet werden. Dies ist besonders vorteilhaft, da die Vorrichtung entsprechend klein dimensioniert werden kann, wodurch sie sich besonders für den Einsatz im Sportbereich eignet.

Beispielsweise kann die Dichtlippe für eine Aufnahme mit einem Durchmesser von 8 mm eine teilweise Veränderung des Durchmessers aufgrund des Ausbauchens von bis zu 1 mm, vorzugsweise 1, 2 mm ausgleichen. Ein solches konstruktiv eingeplantes Ausbauchen der Aufnahme hat zudem den Vorteil, dass die Vorrichtung im blockierten Zustand auch die Gelenkfehlbewegung abdämpft.

Weiterhin ist die Dichtlippe derart konfiguriert, dass dies wieder nach der Verformung rückstellbar ist, so dass sie sich nach adaptiver Verformung, wieder annähernd in den Ausgangszustand (weitgehend eigenständig) zurückstellt, bzw. zurückfedert. Dafür sollte die Dichtlippe entsprechende wie oben beschriebene Elastizitätsmodule aufweisen.

Die Durchlassöffnung verbindet eine erste Kammer mit einer zweiten Kammer im Innenraum der Aufnahme fluidtechnisch.

Die Aufnahme besitzt einen größeren Durchmesser als der Wirkkörper. Der Spalt wird durch die unterschiedlichen Durchmesser von Wirkkörper und Aufnahme gebildet.

Gemäß einer Ausführungsform ist das Füllmedium ab einer um mehr als 15 % vergrößerten Größe des Spalts, bezogen auf die ursprüngliche Größe des Spalts, durch den Spalt strömbar, um einen kritischen Innendruck in der Aufnahme zu vermeiden.

Das Ausbauchen wird somit bewusst nur bis zu einem gewissen Grad kompensiert, d. h. bis ein gewisser (kritischer) Innendruck in der Aufnahme entsteht. Bei einem kritischen Innendruck baucht die Aufnahme in einem solchen Maß auf, dass die Dichtlippe den Spalt nur teilweise abdichtet, um die Entstehung eines kritischen Innendrucks entgegen zu wirken. Im oben geschilderten Beispiel würde sich der Durchmesser beispielweise um mehr als 1 mm bzw. 1, 2 mm vergrößern.

Gemäß einer Ausführungsform ist die Dichtlippe und der Wirkkörper einstückig ausgebildet. Zusammen bilden die Dichtlippe und der Wirkkörper die einstückige Wirkkörperanordnung. Dadurch kann ein Lösen bzw. Abrutschen der Dichtung von dem Wirkkörper vermieden werden und die Sicherstellung der Abdichtung des Spalts zwischen Aufnahme und Wirkkörper verbessert werden.

Darüber hinaus ermöglicht die einstückige Ausbildung eine vereinfachte Montage, was sich wiederum positiv auf die Herstellkosten der Vorrichtung auswirkt.

Gemäß einer Ausführungsform umfasst die Aufnahme eine Öffnung, wobei der Kraftübertragungskörper durch die Öffnung verläuft, und wobei die Dichtlippe an einem der Öffnung der Aufnahme zugewandten Ende des Wirkkörpers angeordnet ist. Der Kraftübertragungskörper ist entlang einer Auszugsrichtung verschiebbar. Die Auszugsrichtung verläuft im Wesentlichen entlang der Längsachse des Wirkkörpers. Diese Anordnung der Dichtlippe am Wirkkörper hat den Vorteil, dass die Dichtlippe neben der Dichtfunktion auch eine Pufferfunktion gegen das Anschlagen des Wirkkörpers auf der Aufnahme bereitstellt.

Die Längsachse ist als eine Achse zu verstehen, die entlang der Auszugsrichtung oder der Mittellinie des Kraftübertragungskörpers verläuft.

Alternativ kann eine zusätzliche Dichtlippe auch an einem der Öffnung gegenüberliegendes Ende des Wirkkörpers angeordnet sein. Dadurch kann die Dichtwirkung weiter erhöht werden und ein Anschlagen des Wirkkörpers an dem der Öffnung genüberliegenden Bereiche der Aufnahme vermieden werden.

In einem Beispiel erstreckt sich die Dichtlippe zumindest teilweise von dem äußeren Umfang des Wirkkörpers in Richtung einer Innenwand der Aufnahme und ist an dieser anliegbar. Dadurch kann ein Anliegen der Dichtlippe in einer Ausgangsstellung, d. h. in der die Aufnahme nicht ausbaucht, sichergestellt werden. Die Dichtlippe ist beispielsweise derart vorgespannt, dass die Dichtlippe in der Ausgangsstellung an der Innenwand der Aufnahme abdichtend anliegt.

Alternativ ist die Dichtlippe nicht vorgespannt, sondern derart ausgestaltet, dass die Dichtlippe in der Ausgangsstellung einen kleinen Spalt zwischen Dichtlippe und Innenwand der Aufnahme zulässt. Wird die Vorrichtung im Bereich der unphysiologischen Geschwindigkeiten aktiviert, wirkt das Füllmedium auf die Dichtlippe, sodass diese auf die Innenwand der Aufnahme gedrückt wird.

Gemäß einer besonders bevorzugten Ausführungsform erstreckt sich die Dichtlippe zumindest teilweise in Richtung der Öffnung der Aufnahme über den Wirkkörper hinaus, um einen Vorsprung in eine Auszugsrichtung des Wirkkörpers zu bilden.

Eine solche Ausrichtung der Dichtlippe hat den Vorteil, dass die Dichtlippe durch das Füllmedium an die Innenwand der Aufnahme gedrückt wird, wenn der Kraftübertragungskörper aus der Vorrichtung in Auszugsrichtung ausgezogen wird. Das Füllmedium wird in einer ersten Kammer der Aufnahme, d. h. der Kammer die der Öffnung der Aufnahme näher ist, komprimiert und fließt zumindest entlang der Innenwand der Aufnahme in entgegengesetzter Richtung zu der Auszugsbewegung des Wirkkörpers. Das Füllmedium drückt somit auf die Dichtlippe, wodurch die Dichtlippe auf die Innenwand der Aufnahme gedrückt wird und die abdichtende Wirkung der Dichtlippe verstärkt.

Weiterhin hat eine solche Anordnung den Vorteil, dass die Dichtlippe puffernd wirkt, wenn der Wirkkörper ein zweites Ende, d. h. das Ende, wo die die Öffnung der Aufnahme angeordnet ist, anschlägt. Dies ist zum Beispiel dann der Fall, wenn die Vorrichtung bei physiologischen Geschwindigkeiten aktiviert bzw. der Wirkkörper auf den Anschlag der Aufnahme gezogen wird. Durch die abdämpfende Wirkung der Dichtlippe kann eine Beschädigung des Wirkkörpers und/ oder der Aufnahme durch den Aufprall auf die Aufnahme vermieden werden.

Besonders bevorzugt ist die Dichtlippe zum Schließen des Spalts zwischen der Wirkkörperanordnung und der Aufnahme schirmartig oder schenkelartig von dem Wirkkörper abspreizbar. Dadurch kann eine vollumfängliche Dichtung des Spalts sichergestellt werden.

Schirmartig ist vorliegend als eine von einer Längsachse des Wirkkörpers weg weisende Ausrichtung im Raum zu verstehen. Die von dem Wirkkörper abgespreitzte Dichtlippe ist in einem Winkel α, β zwischen der Längsachse und einer Senkrechten der Längsachse im Raum angeordnet, d. h. im Winkelbereich von größer 0° und kleiner 90°, vorzugsweise größer 10°C und kleiner 80°C bezogen auf die Längsachse des Wirkkörpers. 0°C ist als eine Ausrichtung der Dichtlippe zu verstehen, die parallel zu der Längsachse des Wirkkörpers verläuft. 90°C ist als eine Ausrichtung der Dichtlippe zu verstehen, die senkrecht zu der Längsachse des Wirkkörpers verläuft. Alternativ kann die Dichtlippe auch entlang der Längsachse des Wirkkörpers und entlang des Umfangs des Wirkkörpers partiell versetzt sein, d. h. z. B. blütenartig am Wirkkörper angeordnet sein. Weiterhin kann der Wirkkörper auch konisch gestaltet sein.

Die von dem Wirkkörper abstehende Dichtlippe kann in verschiedenen Formen ausgestaltet sein. Beispielsweise kann die Dichtlippe geradlinig oder gebogen ausgestaltet sein. Weiterhin kann das Ende der Dichtlippe, das der Innenwand der Aufnahme zugewandt ist, einen größeren Querschnitt aufweisen als der Rest der Dichtlippe. Dadurch kann die Kontaktfläche an der Innenwand für die Abdichtung vergrößert werden. Ein verjüngter Querschnitt der Dichtlippe im anliegenden Bereich an den Wirkkörper ist vorteilhaft, da dadurch die Dichtlippe flexibler ist, und somit adaptiv die Veränderungen des Durchmessers der Aufnahme ausgleichen kann.

Gemäß einer Ausführungsform weist die Dichtlippe ein Elastizitätsmodul im Bereich von 300-700 MPa, vorzugsweise im Bereich von 350-450 MPa, besonders bevorzugt von 420 MPa auf. Dadurch wird eine Dichtlippe bereitgestellt, die sowohl geeignet ist den Spalt zwischen dem Wirkkörper und der Aufnahme gegen das Durchströmen von Füllmedium in der Ausgangsstellung abzudichten als auch die Spaltgrößenveränderungen aufgrund des Ausbauchens oder Spaltgrößentoleranzen auszugleichen.

Besonders bevorzugt ist die Dichtlippe aus einem Polymer, vorzugsweise ein Polymer aus der Klasse der Polyhalogenolefine, besonders bevorzugt Polytetrafluorethylen (PTFE). PTFE ist besonders reibungsarm und besitzt elastische Eigenschaften, die geeignet sind, die Veränderungen des Durchmessers der Aufnahme in Folge eines Ausbauchens auszugleichen.

Gemäß einer weiteren Ausführungsform lässt die die Dichtlippe ab einem bestimmten Schwellwert einer auf den Kraftübertragungskörper wirkenden Kraft bzw. Geschwindigkeit und einem daraus resultierenden bestimmten Druck in der Aufnahme, bevorzugt in einer ersten Kammer in der Aufnahme, ein Strömen von Füllmedium durch den Spalt zu, um einen kritischen Innendruck in der Aufnahme zu vermeiden.

Beispielsweise sind in der Dichtlippe Schlitze oder Klappen eingearbeitet, die ab einem bestimmten Schwellwert einer auf den Kraftübertagungskörper ausgeübten Zugkraft ausgedehnt werden und das Füllmedium durch die Dichtlippe passieren lässt. Unter dem bestimmten Schwellwert sind die Schlitze und Klappen durch die Eigenspannung der Dichtlippe verschlossen. Dadurch wirkt die Dichtlippe als ein Überdruckventil. Dies hat den Vorteil, dass in der Aufnahme kein Überdruck entsteht. In einem Beispiel kann die Dichtlippe konfiguriert sein, um beispielsweise eine Gegenkraft von 800 N zu gewährleisten, unabhängig davon mit welcher Kraft der Kraftübertragungskörper aktiviert wird. Wenn der Kraftübertragungskörper beispielsweise mit einer Zugkraft von 2000 N aktiviert wird, wirkt die Dichtlippe als Überdruckventil indem eine gewisse Menge an Füllmedium über die ab einem bestimmten Schwellwert durchlässige Dichtlippe durch den Spalt durchgelassen wird, um stets eine Gegenkraft von beispielsweise 800 N bereitzustellen. Dadurch können die Lebensdauer und die Sicherheit der Aufnahme, z. B. Vermeidung von Platzen der Aufnahme, verbessert werden.

In einem Beispiel kann der Wirkkörper einen Anschlag aufweisen, der die Bewegung in Richtung der Innenseite der Aufnahme abhängig von einem bestimmten Druck begrenzt. Dadurch liegt die Dichtlippe ab einem bestimmten Druck nicht an der Innenseite der Aufnahme an, sodass ein geringer Strom an Füllmedium durch den Spalt passieren kann, um das Aufbauen eines Überdrucks zu vermeiden.

Des Weiteren kann dadurch ein weiches Abbremsen der Gelenksfehlbewegung gewährleistet werden. Dies ist physiologisch gesehen günstiger als ein kompletter, abrupter Stopp der Vorrichtung.

Gemäß einer weiteren Ausgestaltung ist um eine Öffnung der Aufnahme, ein Endanschlag an der Aufnahme angeordnet, um ein direktes Aufprallen des Wirkkörpers auf die Aufnahme zu vermeiden.

Der Endanschlag ist beispielsweise ein elastischer, weicher Kunststoff, der als ein Pufferelement oder Polster an der Innenseite der Aufnahme um die Öffnung der Aufnahme angeordnet ist. Der Endanschlag kann auch im Zweikomponenten-Spritzgussverfahren in die Aufnahme integriert werden.

In einem weiteren Beispiel kann die Dichtlippe und der Endanschlag in Kombination eine puffernde Funktion bereitstellen. Dies ist besonders dann wichtig, wenn die Vorrichtung nicht blockiert, d. h. die Bewegungen im physiologischen Bereich sind. Dann nämlich kann der Wirkkörper bis zum Anschlag der Aufnahme gelangen. Ohne einen Puffer besteht die Gefahr, dass Teile der Vorrichtung, insbesondere der Wirkkörper bzw. die Dichtlippe und die Aufnahme, durch den Aufprall des Wirkkörpers auf die Aufnahme beschädigt werden. Weiterhin kann die Dichtlippe auch an einem der Öffnung der Aufnahme gegenüberliegendes Ende, angebracht sein, um dort ein Aufprall-Dämpfungselement bzw. ein Pufferelement zu bilden.

Der zweite Wirkkörper ist in Bezug auf die Längsachse der Aufnahme näher an dem zweiten Ende der Aufnahme angeordnet, wobei der erste Wirkkörper in Bezug auf die Längsachse näher an dem ersten Ende angeordnet ist.

In einer bevorzugten Weiterbildung umfasst der zweite Wirkkörper mindestens einen Aufnahmeraum in der relativen Verschieberichtung, wobei der Aufnahmeraum mit dem ersten Wirkkörper in Bezug auf die relative Verschieberichtung unabhängig von einer relativen Verschiebung des ersten Wirkkörpers und des zweiten Wirkkörpers zu einander überlappt.

Dadurch ist es möglich stets einen Kontakt, d. h. eine Führung des ersten Wirkkörpers am zweiten Wirkkörper bereitzustellen. Folglich besteht eine Überlappung des Aufnahmeraums des zweiten Körpers mit dem ersten Körper sowohl wenn sich die Vorrichtung in einer Ausgangsposition befindet, in welcher keine äußere Kraft auf die Vorrichtung und insbesondere den ersten und den zweiten Wirkkörper wirkt, als auch in einer Blockierposition, in welcher der erste Wirkkörper auf dem zweiten Wirkkörper aufsitzt und dabei die Durchlassöffnung verschließt

Eine Überlappung des ersten Wirkkörpers und des zweiten Wirkkörpers in einer relativen Verschieberichtung ermöglicht es die beiden Wirkkörper zu führen, wenn sie aufgrund einer äußeren Krafteinwirkung relativ zueinander bewegt werden. Dadurch kann die Wahrscheinlichkeit erhöht werden, dass durch ein Kontaktieren der beiden Wirkkörper, welches das Blockieren der Durchlassöffnung zur Folge hat, die Durchlassöffnung vollständig geschlossen ist. Dies trägt zu einem gleichmäßigen Verhalten der Vorrichtung bei.

Die relative Verschieberichtung bezeichnet die Richtung, in der der erste Wirkkörper und der zweite Wirkkörper durch eine äußere Krafteinwirkung und/oder den Kraftübertragungskörper relativ zueinander bewegt werden können.

In einem Beispiel besitzt die Aufnahme einen Durchmesser im Bereich von 4-15 mm. Die Wahl des Durchmessers hängt vom Anwendungsfall ab. Beispielsweise werden in Sportgeräten Vorrichtungen mit einem Durchmesser von 15 mm eingesetzt. In Einsatzgebieten wie in der Bekleidungsindustrie, z. B. Schuhe, werden beispielsweise Vorrichtungen mit einem Durchmesser von 4mm eingesetzt. Vorrichtungen mit derartigen kleinen Durchmessern können beispielsweise zum Einweben bzw. Stricken in Textilien eingesetzt werden. Desto kleiner der Durchmesser der Aufnahme ist, desto besser ist der Tragekomfort der Vorrichtung am Körper bzw. desto kleiner ist der Raumbedarf der Vorrichtung bei der Anbringung an Sportgeräten.

Die Dichtlippe ermöglicht es, die Vorrichtung entsprechend klein zu dimensionieren, wodurch sie sich besonders für den Einsatz im Sportbereich eignet. Alternativ können die Vorrichtungen auch für den Arbeitsschutz, im Militärbereich oder allgemein für die Sturzprävention eingesetzt werden.

Bevorzugt besitzt die Aufnahme eine Wandstärke zwischen 1-3 mm, besonders bevorzugt im Bereich zwischen 1, 5-2, 5 mm, weiter besonders bevorzugt von 2 mm.

Gemäß einer weiteren Ausgestaltung ist ein Spaltmaß zwischen dem ersten Wirkkörper und der Aufnahme unterschiedlich zu einem Spaltmaß zwischen dem zweiten Körper und der Aufnahme.

Gemäß einer Ausführungsform ist die Dichtlippe durch ein Zweikomponenten Spritzgussherstellungsverfahren an den Wirkköper angespritzt, wobei zumindest die Dichtlippe ein Polymer, insbesondere aus der Klasse der Polyhalogenolefine, vorzugsweise Polytetrafluorethylen (PTFE), aufweist. PTFE ist besonders reibungsarm und besitzt elastische Eigenschaften, die geeignet sind, die Veränderungen des Durchmessers der Aufnahme auszugleichen. Des Weiteren besitzt PTFE eine hohe Reproduzierbarkeit im Herstellungsprozess. Alternativ kann der Wirkkörper derart gestaltet sein bzw. aus einer Komponente gespritzt werden, dass der Wirkkörper die Dichtlippe an sich ausbildet und sich je nach wirkender Kraft und Geschwindigkeit adaptiv wölbt, bzw. nicht ausprägt.

In einer bevorzugten Weiterbildung ist die Aufnahme eine Hohlfaser. Dies ist beispielsweise vorteilhaft bei der Integration der Vorrichtung in Textilprodukte, wie zum Beispiel Bandagen, Pelotten, Handschuhe, Schuhe, Socken und dergleichen. Das Wirkprinzip auf welchem die Vorrichtung beruht, ermöglicht eine besonders kleine Bauweise. Integriert in eine Hohlfaser ist so eine Vorrichtung zur Bewegungsbegrenzung möglich, welche sich insbesondere für den Sportbereich eignet.

Die Vorrichtung zur Stabilisierung von Körpergelenken und/oder Sportgeräten ermöglicht eine Ausgestaltung der Aufnahme mit elastischen Eigenschaften. Ein Ausbauchen der Aufnahme bei hoher Druckbelastungen der Vorrichtung und ein damit einhergehender Druckverlust wird durch die Dichtlippe kompensiert, da diese sich an den veränderten Querschnitt der Aufnahme beim Ausbauchen angleicht.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1a: schematisch eine Schnittansicht einer Vorrichtung zur Stabilisierung von Bewegungen zweier sich relativ zueinander bewegbarer Teile eines Körperbereichs und/oder eines Sportgeräts gemäß einer Ausführungsform,
- Figur 1b: schematisch eine Detailansicht der Schnittansicht der Vorrichtung aus Figur 1a im nicht blockierten Zustand der Vorrichtung
- Figur 1c: schematisch eine Detailansicht der Schnittansicht der Vorrichtung aus Figur 1a im blockierten Zustand,
- Figur 1d: schematisch eine perspektivische Ansicht der Vorrichtung aus Figur 1a,
- Figur 2: schematisch eine Schnittansicht einer Vorrichtung zur Stabilisierung von Bewegungen zweier sich relativ zueinander bewegbarer Teile eines Körperbereichs und/oder eines Sportgeräts gemäß einer weiteren Ausführungsform,
- Figur 3a: eine perspektivische Ansicht des zweiten Wirkkörpers der Vorrichtung aus Figur 2,
- Figuren 3b und 3c: schematisch Seitenansichten des zweiten Wirkkörpers der Vorrichtung aus Figur 2,
- Figuren 4a-4g: perspektivische Ansichten und schematische Schnittansichten verschiedener Ausführungsformen der Dichtlippe,
- Figur 5: schematisch eine Schnittansicht einer Vorrichtung zur Stabilisierung von Bewegungen zweier sich relativ zueinander bewegbarer Teile eines Körperbereichs und/oder eines Sportgeräts mit einem Endanschlag, und
- Figur 6: schematisch eine Schnittansicht einer Vorrichtung zur Stabilisierung von Bewegungen zweier sich relativ zueinander bewegbarer Teile eines Körperbereichs und/oder eines Sportgeräts gemäß einer weiteren Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet.

Um Redundanzen zu vermeiden, wird auf eine wiederholte Beschreibung dieser Elemente in der nachfolgenden Beschreibung teilweise verzichtet.

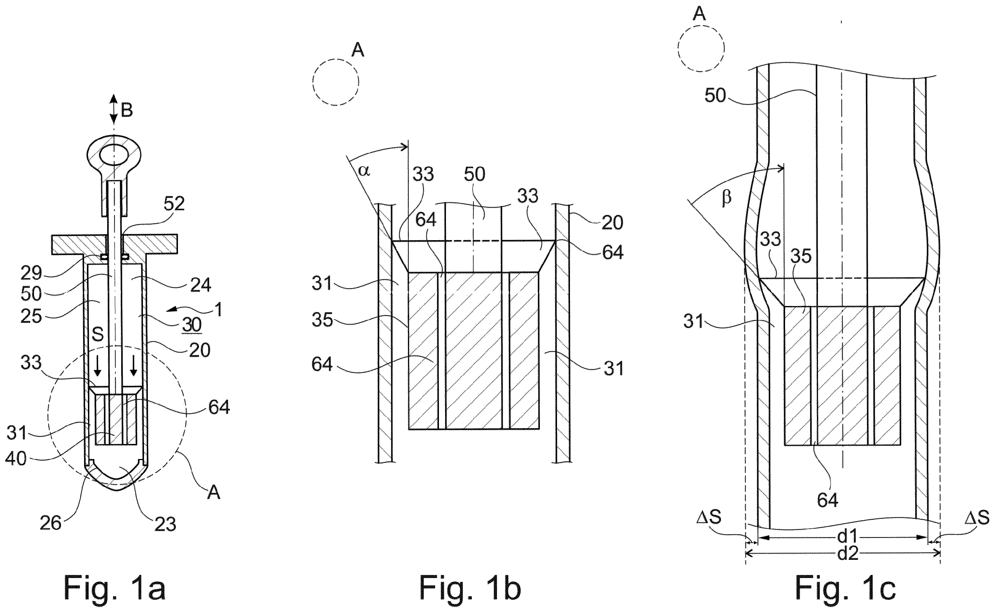

Nachfolgend wird eine Vorrichtung 1 zur Stabilisierung von Bewegungen zweier sich relativ zueinander bewegbarer Teile eines Körperbereichs und/oder eines Sportgeräts anhand der Figuren 1a bis 1c beschrieben. Die Vorrichtung umfasst eine an einem ersten Teil eines Körperbereichs und/oder eines Sportgeräts fixierbare Aufnahme 20, wobei die Aufnahme 20 mit einem Füllmedium 30 gefüllt ist, und mindestens eine Wirkkörperanordnung mit einem Wirkkörper, der in der Aufnahme 20 verschiebbar aufgenommen ist und mit dem Füllmedium interagieren kann, einen an einem zweiten Teil desselben Körperbereichs und/oder desselben Sportgeräts fixierbaren Kraftübertragungskörper 50 zum Übertragen einer äußeren Kraft auf den Wirkkörper 35, wobei der Wirkkörper 35 mindestens eine Durchlassöffnung 64 umfasst, durch welche Füllmedium strömen kann. Die Wirkkörperanordnung weist eine Dichtlippe 33 zum Abdichten eines Spalts 31 zwischen einer Innenseite der Aufnahme 20 und einem seitlichen Bereich einer Außenseite der Wirkkörperanordnung 35 auf, wobei die Dichtlippe 33 an einer Außenseite des Wirkkörpers 35 angeordnet ist.

Der Wirkkörper 35 unterteilt den Innenraum 25 der Aufnahme 20 in eine erste Kammer 23 und eine zweite Kammer 24. Der Kraftübertragungskörper 50 ist an dem Wirkkörper 35 befestigt, welcher längs durch die zweite Kammer 24 verläuft und an einem zweiten Ende 22 der Aufnahme 20 aus diesem heraustritt. Das in der Figur 1a gezeigte freie Ende des Kraftübertragungskörpers 50 kann mit einem Körperteil eines Anwenders (nicht gezeigt) mittels entsprechender Anbindungselemente verbunden werden oder an einem Sportgerät verbunden sein, um eine von dem Körperteil oder dem Sportgerät ausgehende Zugkraft über den Kraftübertragungskörper 50 auf den Wirkkörper 35 zu übertragen. Die Aufnahme 20 umfasst ein erstes Anbindungselement zur Kraftübertragung zwischen der Aufnahme 20 und dem ersten Körper (nicht gezeigt) und der Kraftübertragungskörper 50 umfasst ein zweites Anbindungselement zur Kraftübertragung zwischen dem Kraftübertragungskörper 50 und dem zweiten Körper (nicht gezeigt). Das erste Anbindungselement ist zur Kraftübertragung zwischen der Aufnahme 20 und dem ersten Körper (nicht gezeigt) im Bereich der Aufnahmeöffnung angeordnet. In einem Beispiel ist das erste Anbindungselement als Flansch ausgestaltet.

Das erste Anbindungselement dient als Schnittstelle zur Kraftübertragung zwischen dem ersten vorrichtungsfremden Körper und der Vorrichtung 1. An dem freiliegenden Teil des Kraftübertragungskörpers 50 ist das zweite Anbindungselement angeordnet, dass als Schnittstelle zur Kraftübertragung zwischen einem zweiten vorrichtungsfremden Körper und der Vorrichtung 1 dient. Somit ergibt sich im Belastungsfall ein Kraftfluss zwischen dem ersten Anbindungselement, dem Füllmedium 30, dem Kraftübertragungskörper 50 und dem zweiten Anbindungselement.

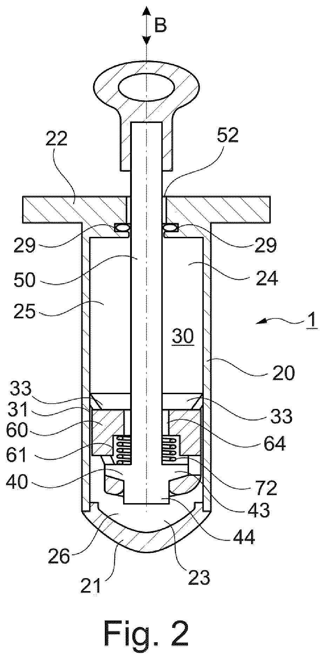

Die Aufnahme 20 ist im Bereich eines ersten Endes 21, siehe Fig. 2, durch einen Verschluss 26 verschlossen, so dass in der Aufnahme 20 befindliches Füllmedium gehalten werden kann.

Im Bereich des zweiten Endes 22, siehe Fig. 2, der Aufnahme 20 ist ein Dichtkörper 29 angeordnet, welcher den Innenraum des Aufnahme 20 gegenüber dem Kraftübertragungskörper 50 abdichtet. Dabei weist die Aufnahme 20 eine Öffnung 52 auf, durch welche der Kraftübertragungskörper 50 aus dem Innenraum der Aufnahme 20 heraustritt.

Der Wirkkörper 35 kann mittels des Kraftübertragungskörpers 50 durch das Füllmedium in Richtung des zweiten Endes 22 bewegt werden. Dabei ist der Aufnahme 20 mit einer ersten Körper- oder Sportgerätestelle verbunden und der Kraftübertragungskörper 50 mit einer zweiten Körper- oder Gerätestelle verbunden, wobei sich die erste Körper- oder Gerätestelle und die zweite Körper- oder Gerätestelle relativ zueinander bewegen können.

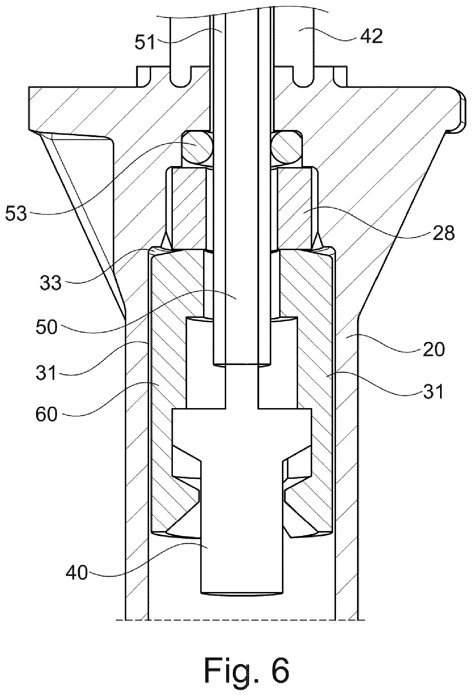

Die Vorrichtung 1 kann anwendungsspezifisch dimensioniert werden, so dass die Vorrichtung 1 physiologische Bewegungen des Anwenders zulässt. Wird der Wirkkörper 35 im Rahmen einer physiologischen Bewegung mittels des Kraftübertragungskörpers 50 in Richtung des zweiten Endes bewegt, kann wie hier gezeigt Füllmedium durch die Durchlassöffnung 64 zwischen der Aufnahme 20 und dem Wirkkörper 35 oder durch ein in dem Wirkkörper 35 angeordnetes Ventil (siehe Fig. 2) von der zweiten Kammer 24 in die erste Kammer 23 strömen. Die Fließgeschwindigkeit des Füllmediums hängt entscheidend von der Querschnittfläche senkrecht zu einer Auszugrichtung B der Aufnahme 20 und des Wirkkörpers 35 ab. Diese zur Strömung für das Füllmedium bereitstehende Querschnittfläche wird auch als hydraulischer Durchmesser bezeichnet und ist letztendlich für das reaktive Verhalten der Vorrichtung bei äußerer Krafteinwirkung entscheidend. So kann durch die Wahl des hydraulischen Durchmessers der Widerstand festgelegt werden, den die Vorrichtung 1 äußeren Kräften bzw. einwirkenden Geschwindigkeiten entgegenbringt. Die Pfeile stellen dabei die Strömungsrichtung S des Füllmediums dar. Wirkt keine Kraft mehr über den Kraftübertragungskörper 50 auf den Wirkkörper 35, so kann der Körper 35 mittels eines Rückstellelements42, siehe Fig. 6, wieder in die Ausgangsposition zurückbewegt werden, wobei das Füllmedium 30 von der ersten Kammer 23 in die zweite Kammer 24 strömt.

Werden hingegen unphysiologische Kräfte, das heißt für das entsprechend zu stabilisierende Körperteil oder Bauteil kritische Kräfte, in die Vorrichtung eingeleitet, ist aufgrund des hydraulischen Durchmessers eine Relativbewegung zwischen dem Wirkkörper 35 und der Aufnahme 20 nur noch unter sehr hohem Kraftaufwand möglich. Bei dem Füllmedium 30 handelt es sich um newtonsche Fluide, wie zum Beispiel Silikonöl. Alternativ können auch dilatante Fluide als Füllmedium verwendet werden. Darüber hinaus kann auch ein Kunststoff verwendet werden.

Wie in Fig. 1b und Fig. 1c in einem detaillierten Ausschnitt von Fig. 1a gezeigt, weist die Wirkkörperanordnung, d. h. der Wirkkörper 35 und die Dichtlippe 33, einen an der Innenseite der Aufnahme 20 anliegenden Bereich der Dichtlippe auf, der in einem Winkel (α, β) zu einem seitlichen Bereich der Außenseite des Wirkkörpers verläuft, wobei der Winkel (α, β) veränderlich ist, um Größenschwankungen (Δ S) des Spalts 31 zwischen der Innenseite der Aufnahme 20 und dem seitlichen Bereich der Außenseite der Wirkkörperanordnung ausgleichen zu können und den Spalt 31 auch bei derartigen Größenschwankungen abzudichten.

Fig. 1b zeigt einen detaillierten Ausschnitt der Vorrichtung aus Fig. 1a. Hier ist gezeigt, dass die Dichtlippe 33 an der Innenwand der Aufnahme 20 in einem Winkel α in Bezug auf die Längsachse des Wirkkörpers 35 anliegt, um ein Strömen des Füllmediums durch den Spalt 31 zu unterbinden, d. h. der Spalt 31 wird durch die Dichtlippe 33 im Wesentlichen abgedichtet bzw. überbrückt. Die Dichtlippe 33 und der Wirkkörper 35 können wie hier gezeigt einstückig ausgestaltet sein und die Wirkkörperanordnung bilden. Dadurch kann das Lösen der Dichtlippe 33 von dem Wirkkörper 35 vermieden werden. Alternativ können Dichtlippe und der Wirkkörper auch mehrteilig ausgebildet sein.

In der in den Figuren 1a bis 1c gezeigten Ausgestaltung erstreckt sich die Dichtlippe 33 in Richtung der Öffnung 52 der Aufnahme 20 über den Wirkkörper 35 hinaus, um einen Vorsprung in eine Auszugsrichtung des Wirkkörpers 35 zu bilden. Eine solche Ausrichtung der Dichtlippe 33 hat den Vorteil, dass die Dichtlippe 33 durch das Füllmedium an die Innenwand der Aufnahme 20 gedrückt wird, wenn der Kraftübertragungskörper 50 aus der Vorrichtung in Auszugsrichtung B ausgezogen wird. Das Füllmedium wird in der zweiten Kammer 24 der Aufnahme komprimiert und das Füllmedium fließt zumindest entlang der Innenwand der Aufnahme 20 in entgegengesetzter Richtung zu der Auszugsrichtung, d. h. auf den Wirkkörper 35 zu. Das Füllmedium drückt somit auf die Dichtlippe 33, wodurch die Dichtlippe 33 auf die Innenwand der Aufnahme 20 gedrückt wird und die abdichtende Wirkung der Dichtlippe 33 verstärkt.

Fig. 1c zeigt die Dichtlippe 33 in einem Zustand, in dem die Aufnahme 20 aufgrund einer Druckerhöhung im Inneren der Aufnahme auswölbt bzw. ausbaucht. Im Inneren der Aufnahme der Vorrichtung können durch die kritischen Verschiebegeschwindigkeiten und der daraus resultierenden Kräfte auf den Wirkkörper 35 sehr hohe Drücke, bis zu 280 bar entstehen. Die Aufnahme ist aus einem elastischen Material, das bei hoher Druckbelastung nach außen auswölbt bzw. ausbaucht, d. h. die Wände der Aufnahme wölben sich bogenartig nach außen.

Durch das Ausbauchen der Aufnahme 20 wird der Querschnitt der Aufnahme 20 partiell vergrößert. Die Dichtlippe 33 ist jedoch derart elastisch, dass kein Füllmedium durch den Spalt 31 strömt, auch wenn sich der Querschnitt der Aufnahme 20 d. h. der Durchmesser der Aufnahme verändert. Wie in Fig. 1c gezeigt breitet sich die Dichtlippe nach außen aus, d. h. in Richtung der Innenwand der Aufnahme, um die Auswölbung auszugleichen. In Bezug auf die Längsachse des Wirkkörpers steht die Dichtlippe nun nicht mehr im Winkel α im Raum, sondern im Winkel β, wobei β größer als α ist. Dadurch entsteht im Inneren der Aufnahme 20, insbesondere in der zweiten Kammer 24, kein Druckverlust, so dass die Funktion der Vorrichtung 1 gewahrt werden kann.

Die umlaufende Dichtlippe hat somit den Vorteil, dass sie einen variierenden Spalt 31 oder Abstand zwischen dem Wirkkörper 35 und einer Innenwand der Aufnahme 20 ausgleicht, also dass sie sich bei einer Auswölbung oder Ausbauchung der Aufnahme 20 mit verformt und somit den Druck in der Aufnahme 20 aufrechterhalten kann, wenn die Vorrichtung 1 blockiert.

Gemäß einer besonders bevorzugten Ausgestaltung kann der veränderliche Winkel (α, β) eine Größenschwankung des Spalts 31 von bis zu 15%, bezogen auf die ursprüngliche Größe des Spalts 31 ausgleichen. Mit anderen Worten ist die Dichtlippe 33 derart adaptiv gestaltet, dass die Dichtlippe 33 auch bei verändertem Durchmesser d2 der Aufnahme und einer daraus resultierenden Spaltgrößenschwankung (Δ S) zwischen der Aufnahme 20 und dem Wirkkörper abdichten kann. Fig. 1c zeigt den ursprünglichen Durchmesser d1 und den veränderten Durchmesser d2 der Aufnahme 20 nach dem Ausbauchen, wobei d2-d1= Δ S (max) entspricht, d. h. der maximalen Größenschwankung des Spalts 31 entlang der Auswölbung der Aufnahme 20, die die Dichtlippe 33 abdichten muss.

Eine solche adaptive Dichtlippe hat den Vorteil, dass die Aufnahme elastisch ausgestaltet werden kann ohne dabei die Funktionsfähigkeit der Vorrichtung zu beinträchtigen. Beispielsweise, wie in Fig. 1c gezeigt, kann die Dichtlippe für eine Aufnahme mit einem Durchmesser vom 8 mm eine teilweise Veränderung des Durchmessers aufgrund des Ausbauchens von bis zu 1mm, vorzugsweise 1, 2 mm, ausgleichen.

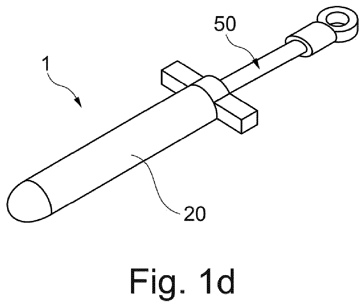

Fig. 1a zeigt die Aufnahme 20 mit einer Öffnung 52, wobei der Kraftübertragungskörper 50 durch die Öffnung 52 verläuft, und wobei die Dichtlippe 33 an einem der Öffnung 52 der Aufnahme 20 zugewandten Ende 22 des Wirkkörpers 35 angeordnet ist. Der Kraftübertragungskörper 50 ist entlang einer Auszugsrichtung B verschiebbar. Die Auszugsrichtung B verläuft im Wesentlichen entlang der Längsachse des Wirkkörpers 1. Wie in Fig. 1 b und Fig. 1c im Detail beispielhaft gezeigt erstreckt sich die Dichtlippe 33 in Richtung der Öffnung 52 der Aufnahme 20 über den Wirkkörper 35 hinaus, um einen Vorsprung in eine Auszugsrichtung des Wirkkörpers 35 zu bilden.

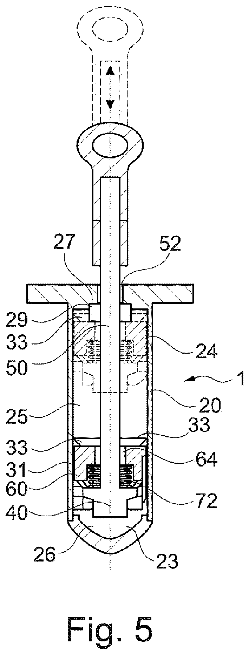

Dies hat den Vorteil, dass die Dichtlippe abdämpfend wirkt, wenn der Wirkkörper an das zweite Ende anschlägt. Dies ist zum Beispiel dann der Fall, wenn die Vorrichtung bei physiologischen Geschwindigkeiten aktiviert wird (siehe Fig. 5). Durch die abdämpfende Wirkung der Dichtlippe kann eine Beschädigung des Wirkkörpers durch den Aufprall auf die Aufnahme vermieden werden.

In Fig. 1d ist eine perspektivische Ansicht der Vorrichtung 1 gezeigt. Aus der Aufnahme 20 ragt der Kraftübertragungskörper 50 heraus. Dabei kann die Aufnahme 20 an einem Körperpunkt eines Anwenders oder Sportgeräts wie zum Beispiel eines Sportschuhs befestigt werden. Das Ende des Kraftübertragungskörpers 50, welches außerhalb der Vorrichtung 20 liegt, kann an einem zweiten Körperpunkt befestigt werden. Die Aufnahme 20 ist zylinderförmig ausgebildet. Alternativ kann die Aufnahme auch quaderförmig oder oval ausgebildet sein.

Fig. 2 zeigt eine weitere bevorzugte Ausgestaltung der Vorrichtung 1. Die Wirkkörperanordnung weist dabei einen ersten Wirkkörper 40 und einen zweiten Wirkkörper 60 auf, wobei der erste Wirkkörper 40 und der zweite Wirkkörper 60 in der Aufnahme 20 verschiebbar angeordnet sind und mit dem Füllmedium 30 interagieren können, wobei ein Kraftübertragungskörper 50 die äußere Kraft auf den ersten Wirkkörper 40 überträgt, wobei der zweite Wirkkörper 60 über ein Kopplungselement 72 elastisch mit dem ersten Wirkkörper 40 gekoppelt ist, wobei der zweite Wirkkörper 60 und/oder der erste Wirkkörper 40 mindestens eine Durchlassöffnung 64 aufweist, durch welche das Füllmedium 30 strömen kann. Die Durchlassöffnung 64 in dem zweiten Wirkkörper 60 stellt einen hydraulischen Durchmesser für das Füllmedium bereit, durch welchen das Füllmedium 30 fließen kann, solange zwischen dem ersten Wirkkörper 40 und dem zweiten Wirkkörper 60 ein Abstand besteht.

Darüber hinaus bildet der erste Wirkkörper 40 einen Ventilkörper und der zweite Wirkkörper 60 einen Ventilsitz, wobei die Dichtlippe 33 an dem zweiten Wirkkörper 60 angeordnet ist. Dadurch kann ein Fluss des Mediums durch die Durchlassöffnung 64 in Abhängigkeit von der Ventilstellung zugelassen oder unterbunden werden. Der Fluss des Füllmediums kann durch die Durchlassöffnung 64 in Abhängigkeit von der Ventilstellung zugelassen oder unterbunden werden. Auf den ersten Wirkkörper 40 einwirkende äußere Kräfte können über das Kopplungselement 72 auf den zweiten Wirkkörper 60 übertragen werden. Entsprechend ist der erste Wirkkörper 40 dazu in der Lage, den zweiten Wirkkörper 60 mittels des Kopplungselements 72 durch das Füllmedium zu schieben und/oder zu ziehen. Der zweite Wirkkörper 60 ist in Bezug auf die Längsachse der Aufnahme näher an dem zweiten Ende 22 der Aufnahme 20 angeordnet, wobei der erste Wirkkörper 40 in Bezug auf die Längsachse näher an dem ersten Ende 21 angeordnet ist.

Der zweite Wirkkörper 60 weist einen Aufnahmeraum 61 auf, der sich hin zu dem ersten Wirkkörper 40 erstreckt und mit diesem überlappt. Der Aufnahmeraum 61 ist dazu konfiguriert, den ersten Wirkkörper 40 zu hintergreifen. Dabei ist der erste Wirkkörper 40 teilweise in dem Aufnahmeraum 61 aufgenommen.

Gemäß der vorliegenden Ausführungsform ist der erste Wirkkörper 40 stufenförmig aufgebaut. So weist der erste Wirkkörper 40 einen ersten Abschnitt 43 auf, der in dem Aufnahmeraum 61 des zweiten Wirkkörpers 60 verschiebbar aufgenommen ist und einen gegenüber dem ersten Abschnitt 43 im Durchmesser verjüngten zweiten Abschnitt 44, der sich aus dem Aufnahmeraum heraus in Richtung der Bewegungsrichtung B erstreckt. Der Aufnahmeraum 61 ist derart konfiguriert, dass er den ersten Abschnitt 43 des ersten Wirkkörpers 40 hintergreift und eine Führung in Bewegungsrichtung B für den zweiten Abschnitt 44 bildet. Auf diese Weise lässt sich der erste Wirkkörper 40 gegenüber dem zweiten Wirkkörper 60 zentrieren, sodass stets ein Verschließen der Durchlassöffnung 64 sichergestellt werden kann, wenn der erste Wirkkörper 40 und der zweite Körper 60 eine geschlossene Ventilstellung einnehmen.

Dabei ist das Kopplungselement 72 derart konfiguriert, dass es beim Einwirken einer äußeren Kraft auf den ersten Wirkkörper, im Bereich einer physiologischen Geschwindigkeit, eine Kraft auf den zweiten Wirkkörper 60 überträgt, so dass dieser gemeinsam mit dem ersten Wirkkörper 40 durch das Füllmedium bewegt werden kann.

Führt die über den ersten Wirkkörper 40 und das Kopplungselement 72 auf den zweiten Wirkkörper wirkende Kraft zu kritischen relativen Verschiebegeschwindigkeiten in der Vorrichtung, das heißt zu unphysiologischen Geschwindigkeiten, gibt das Kopplungselement 72 nach, wodurch sich der erste Wirkkörper 40 auf den zweiten Wirkkörper 60 zubewegt. Dadurch verringert sich der hydraulische Durchmesser, bis das durch die beiden Wirkkörper gebildete Ventil geschlossen ist.

Wenn kein hydraulischer Durchmesser mehr zur Verfügung steht, durch den das Füllmedium fließen kann, können der erste Wirkkörper 40 und der zweite Wirkkörper 60 nicht weiter durch das Füllmedium bewegt werden. Die Vorrichtung 1 blockiert.

Die Dichtlippe 33 verhindert somit, dass das Füllmedium über den Spalt 31strömen kann und stellt dies auch bei einem Ausbauchen der Aufnahme 20 durch die adaptive Ausgestaltung der Dichtlippe sicher (siehe Fig. 1c).

Durch den Aufprall des ersten Wirkkörpers 40 auf den Wirkkörper 60 kann der der zweite Wirkkörper 60 verformt werden. Es können partiell bis zu 1000 bar Druck auf den zweiten Wirkkörper an der Kontaktstelle von erstem Wirkkörper 40 zu zweitem Wirkkörper 60 wirken. Daher kann in einer weiteren Ausgestaltung der zweite Wirkkörper 60 über eine vorteilhafte Bauteilauslegung verfügen, welche in Form einer entsprechenden Materialpaarung (harte Komponente/weiche Komponente) über einen zwei Komponenten Spritzguss realisiert werden kann. Dabei kann der Bereich des zweiten Wirkkörpers, der mit dem ersten Wirkkörper in Berührung kommt, aus einem Material mit hoher Festigkeit (z. B. E-Modul 1800MPa) gespritzt werden, wobei die Gegenseite des zweiten Wirkkörpers, d. h. die Seite die die Dichtlippe ausbildet aus einer weichen Komponente mit hoher Elastizität (z. B. E-Modul 300-700 MPa) gespritzt wird.

Alternativ kann auch in den Aufnahmeraum 61 ein Insert/ Einlagebauteil (nicht gezeigt) aus einer harten Komponente wie Metall in dem Aufnahmeraum 61 angeordnet sein.

Durch die entsprechende vorteilhafte Auslegung bzw. durch das Metall-Insert kann verhindert werden, dass der erste Wirkkörper 40, bzw. der erste Abschnitt 43 auf den zweiten Wirkkörper 60 destruktiv drückt bzw. einwirkt.

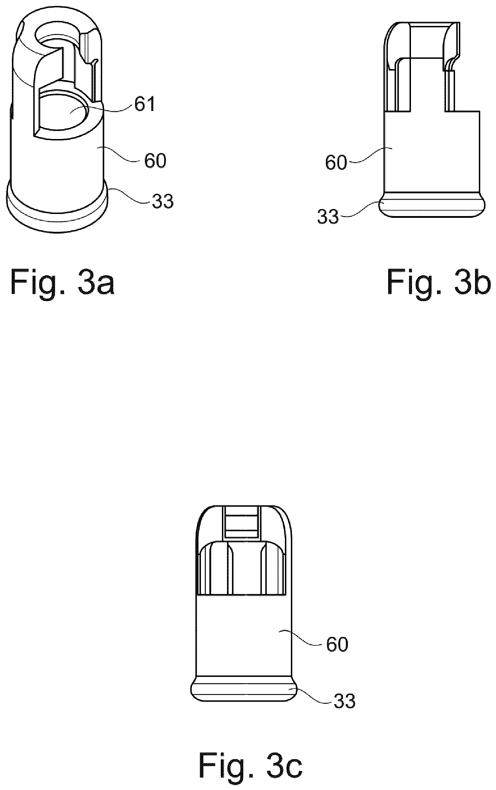

Fig. 3a -Fig. 3c zeigen schematische Ansichten des zweiten Wirkkörpers 60. Der zweite Wirkkörper 60 ist derart gestaltet, dass der Kopplungsübertragungskörper, das Kopplungselement und der erste Wirkköper darin umschlossen sind. Der zweite Wirkköper 60 weist einen käfigartigen Aufnahmeraum auf (siehe auch Fig. 2).

Die Dichtlippe 33 bildet gemeinsam mit dem Wirkkörper 60 eine Wirkkörperanordnung. Gemäß der hier gezeigten Ausgestaltung ist die Dichtlippe 33 zum Schließen des Spalts zwischen dem zweiten Wirkkörper 60 und der Aufnahme schirmartig von dem zweiten Wirkkörper 60 abspreizbar. Dadurch kann eine vollumfängliche Dichtung des Spalts 31 sichergestellt werden.

Die von dem Wirkkörper abgespreitzte Dichtlippe ist in einem Winkel α, β zwischen der Längsachse und einer Senkrechten der Längsachse im Raum angeordnet, d. h. im Winkelbereich von größer 0° und kleiner 90°, vorzugsweise größer 10°C und kleiner 80°C bezogen auf die Längsachse des Wirkkörpers. 0°C ist als eine Ausrichtung der Dichtlippe zu verstehen, die parallel zu der Längsachse des Wirkkörpers verlaufen würde. 90°C ist als eine Ausrichtung der Dichtlippe zu verstehen, die senkrecht zu der Längsachse des Wirkkörpers verlaufen würde.

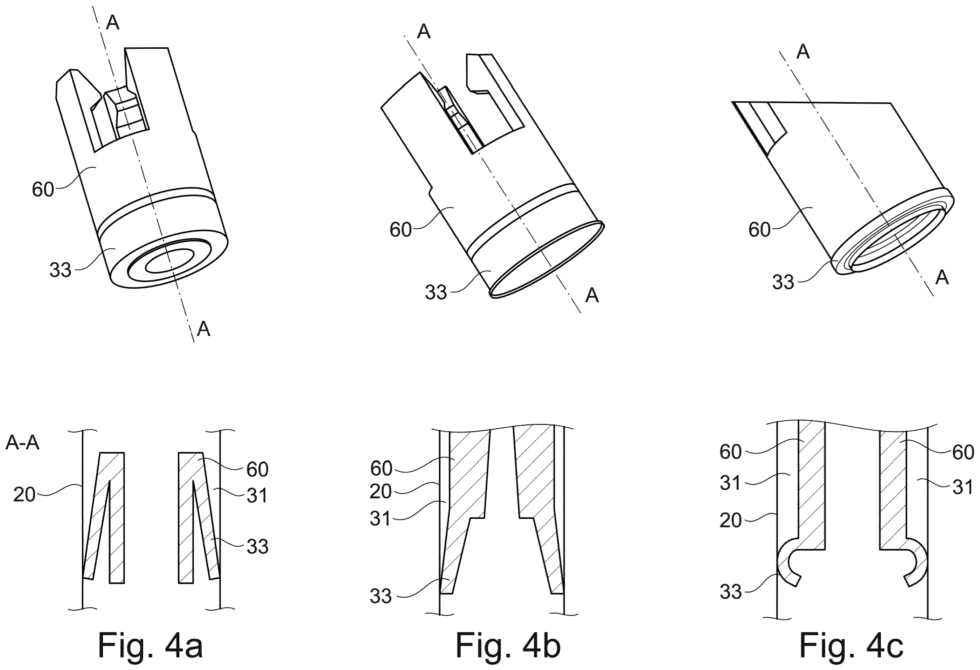

Fig. 4a bis Fig. 4f zeigen weitere alternative Ausgestaltungen der Dichtlippe. Wie hier gezeigt, kann die von dem Wirkkörper abstehende Dichtlippe 33 in verschiedenen Formen ausgestaltet sein. Beispielsweise kann die Dichtlippe geradlinig (siehe Fig. 4a oder Fig. 4b) oder gebogen (siehe Fig. 4c) ausgestaltet sein. Wir hier gezeigt kann die Dichtlippe 33 in unterschiedlicher Weise in den Wirkkörper 35, 60 eingearbeitet sein. In einem bevorzugten Beispiel ist die Dichtlippe 33 mittels eines Zweikomponenten-Spritzgussverfahrens an den Wirkköper 35, 60 angespritzt, wobei zumindest die Dichtlippe ein Polymer, insbesondere aus der Klasse der Polyhalogenolefine, vorzugsweise Polytetrafluorethylen (PTFE), aufweist.

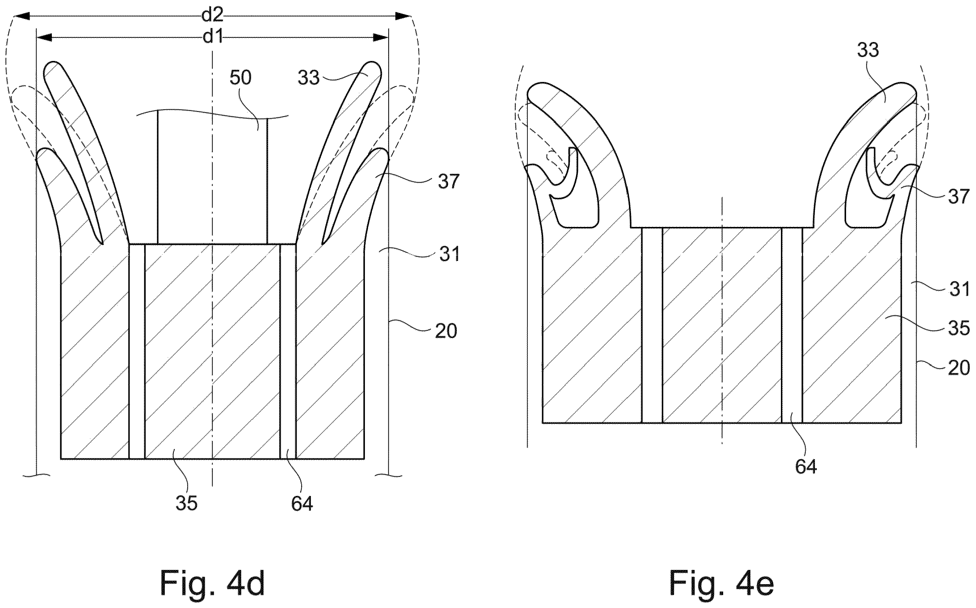

Weiterhin kann wie in Fig. 4d gezeigt, die Dichtlippe 33 durch ein Begrenzungselement 37 in ihrer Bewegung begrenzt werden. Dadurch ist stets ein kleiner Spalt zwischen der Dichtlippe 33 und der Aufnahme 20 bereitstellbar. In einem Beispiel kann das Begrenzungselement 37 derart dimensioniert sein (z. B. ein harter Kunststoff mit einem höheren Elastizitätsmodul als die Dichtlippe), dass eine Ausbreitung der Dichtlippe in Richtung der Innenwand der Aufnahme ab einem bestimmten Druck in der Aufnahme verhindert wird. Im Ausgangszustand liegt die Dichtlippe nicht am Begrenzungselement 37 an. Im ausgebreiteten Zustand, gekennzeichnet durch die gestrichelte Linie, breitet sich die Dichtlippe in Richtung der Innenwand der Aufnahme 20 aus; jedoch nur bis das Begrenzungselement 37 es zulässt. Dadurch kann sichergestellt werden, dass ein geringer Film an Füllmedium durch den Spalt strömen kann, wodurch kritische, hohen Drücke vermieden werden können. Gleichzeitig ist der vorgesehene Spalt derart durch das Begrenzungselement eingestellt, dass kein Druckverlust entsteht und die Vorrichtung immer noch blockiert.

In Fig. 4e ist eine weitere Ausgestaltung eines Begrenzungselements 37 gezeigt. Wie gezeigt kann das Begrenzungselement zusätzlich elastische Eigenschaften besitzen, die die Dichtlippe 33 in den Ausgangszustand zurückstellen kann (siehe gestrichelte Darstellung). Das Rückstellelement kann hierbei beispielsweise federnd fungieren, so dass sich die Dichtlippe nach Belastung umgehend zurückstellt. Gleichzeitig ist das Begrenzungselement 37 derart konfiguriert, dass die Dichtlippe 33 in der Ausbreitung begrenzt werden kann. Dabei gilt wie oben beschrieben, dass die Dichtlippe sich bis zu einem gewissen Druck ausbreiten kann. Sobald ein kritischer Druck in der Aufnahme 20 erreicht wird, begrenzt das Begrenzungselement 37 eine weitere Ausbreitung und stellt damit sicher, dass keine zu hohen Drücke in der Aufnahme 20 entstehen.

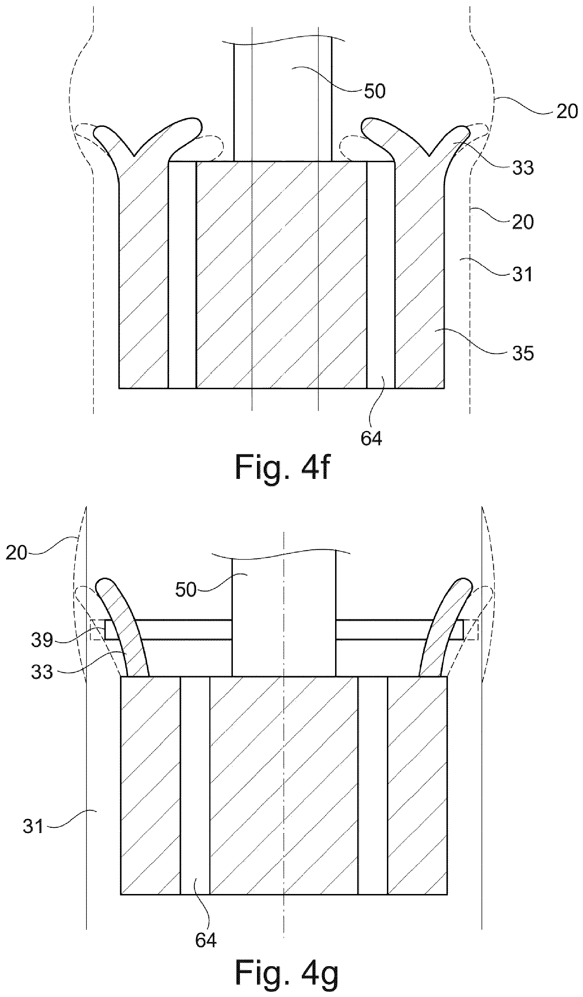

Fig. 4f zeigt eine weitere Ausgestaltung der Dichtlippe 33. In einer weiteren Ausführungsform kann die Dichtlippe 33 auch derart ausgeprägt sein, dass sie (auch) in Richtung der Durchlassöffnung 64 ausgeprägt ist. Dadurch kann der Wirkkörper 35 derart konfiguriert werden, dass die Dichtlippe 33 die Durchlassöffnung 64 im Wirkkörper 35 ventilartig öffnen bzw. schließen kann. Durch die Rückstelleigenschaften der Dichtlippe oder über ein zusätzliches Rückstellelement kann die Öffnung wieder freigegeben werden. Dabei ist die Dichtlippe 33 derart konfiguriert, dass die Dichtlippe 33 neben dem Spalt 31 auch die Durchlassöffnung 64 abdichten kann, d. h. das Strömen von Füllmedium durch die Durchlassöffnung 64 kann im Wesentlichen verhindert werden. Die gestrichelten Linien zeigen die Ausbreitung der Dichtlippe 33, für einen ausgewölbten Zustand der Aufnahme. Durch das zusätzliche Abdichten der Durchlassöffnung 64 kann das Blockieren der Vorrichtung verbessert werden.

Fig. 4g zeigt eine weitere Ausgestaltung, zur Begrenzung der Ausbreitung der Dichtlippe 33. Der Wirkkörper 35 ist hierbei über ein den Wirkkörper umlaufendes Element 39, wie beispielsweise ein Ring oder eine Manschette, versehen, so dass der Weg bzw. der Ausformungsbereich der Dichtlippe begrenzt ist.

Die Manschette 39 ist um die Dichtlippe 33 angebracht. Die Form der Manschette 39 lässt eine Ausbreitung der Dichtlippe 33 nur bis zu voreingestellten Punkt zu. Beispielsweise kann die Manschette derart konzipiert werden, dass die Manschette 39 bis zu einem vorbestimmten Druck (z. B. 160 bar) eine Ausbreitung der Dichtlippe (siehe gestrichelte Linie) zulässt.

Fig. 5 zeigt die Vorrichtung 1 aus Fig. 2 mit einem Endanschlag 27. Der Endanschlag 27 ist im Bereich einer Öffnung 52 der Aufnahme 20 angeordnet, um ein direktes Aufprallen des Wirkkörpers 60 auf die Aufnahme zu vermeiden. Dies ist dann der Fall, wenn die Vorrichtung im Bereich der physiologischen Geschwindigkeiten bewegt wird, z. B. knickt ein Anwender der Vorrichtung bei langsamer Geschwindigkeit bewusst so weit um, dass die maximale Beweglichkeit der Vorrichtung ausgereizt wird. Durch den Endanschlag 27 kann vermieden werden, dass der Wirkkörper 60 unmittelbar auf das zweite Ende 22 der Aufnahme 20 prallt. Mittels des Endanschlags 27 kann somit ein Aufprall des Wirkkörpers auf das zweite Ende 22 der Aufnahme 20 abgedämpft werden.

Gemäß Fig. 5 ist der Endanschlag 27 ein dämpfender, weicher Kunststoff der als ein Pufferelement an der Innenseite der Aufnahme um die Öffnung 52 der Aufnahme 20 angeordnet ist. Das Pufferelement kann beispielsweise ein Polster aus einem Polymer, insbesondere aus der Klasse der Polyhalogenolefine, vorzugsweise Polytetrafluorethylen (PTFE) sein.

Alternativ könnte ein oberer Bereich des Wirkkörpers der dem zweiten Ende der Aufnahme zugewandt ist mit einem dämpfenden, weichen Kunststoff versehen sein. Beispielsweise kann der Wirkkörper mittels eines Zwei-Komponenten-Spritzgussverfahrens hergestellt werden, wobei die Dichtlippe 33 und der obere Bereich aus einem weichen abdämpfenden Material bestehen und der restliche Bereich des Wirkköpers aus einem harten Material besteht.

In einem weiteren Beispiel kann die Dichtlippe 33 und der Endanschlag 27 in Kombination eine optimale Dämpfung des Wirkkörpers bereitstellen.

In einer weiteren Ausführungsform kann die Dichtlippe 33 auch derart ausgeprägt sein, dass sie versetzt zum Endanschlag des Wirkkörpers angeordnet ist. Dadurch kann einem Verschleiß oder gar einer Zerstörung der Dichtlippe bei wiederholtem Aufprallen auf die Aufnahme vorgebeugt werden. Dabei kontaktiert zunächst ein Abschnitt des Wirkkörpers die Aufnahme bzw. den Endanschlag bevor die Dichtlippe auf die Aufnahme trifft.

Fig. 6 zeigt eine Schnittansicht einer Vorrichtung zur Stabilisierung von Körpergelenken, und/ oder Sportgeräten gemäß einer weiteren Ausführungsform mit einem an der Öffnung angeordneten Dichteinsatz 28 und einer Ummantelung 51 des Kraftübertragungskörpers 50.

Der Dichteinsatz 28 ist an der Öffnung der Vorrichtung 1 unter einem die Öffnung abdichtenden O-Ring 53 angeordnet. Der Dichteinsatz 28 ist derart an das Abmaß der Aufnahme 20 an dem oberen Ende der Aufnahme 20 angepasst, dass ein fester Sitz des Dichteinsatzes 28 an der Aufnahme 20 durch eine Kraftschlussverbindung, insbesondere Presspassverbindung zwischen Dichteinsatz 28 und Aufnahme 20, vorliegt. In einem Beispiel kann der Wirkkörper 60 dazu genutzt werden den Dichteinsatz 28 in die Aufnahme 20 einzupressen. Alternativ kann der Dichteinsatz 28 durch eine formschlüssige oder stoffschlüssige Verbindung an die Aufnahme 20 angebracht werden. Beispielsweise kann der Dichteinsatz auch in einem Zweikomponenten-Spritzgussherstellungsprozess bei der Fertigung der Aufnahme direkt an diese angespritzt werden und somit als einteilig mit der Aufnahme hergestellt werden. Durch den Dichteinsatz 28 kann die Dichtung 53 an der Öffnung verbessert werden, da neben der Dichtwirkung des Dichteinsatzes 28 der Dichteinsatz 28 ebenfalls den festen Sitz des O-Rings 53 sicherstellt. Dadurch wird ein Verrutschen des O-Rings 53 vermieden. Gleichzeitig wird der Aufprallschutz für den Wirkkörper und die Dichtlippe verbessert.

Gemäß einer weiteren Ausführungsform kann der Dichteinsatz 28 zusätzlich zu dem oben beschriebenen Endanschlag an der Aufnahme 20 angeordnet sein.

Weiterhin ist dem in Fig. 6 gezeigten Beispiel der Kraftübertragungskörper 50 als ein Seil, insbesondere ein Drahtseil (z.B. ein Bautenzug) ausgestaltet, der zumindest für den mit dem Füllmedium in Kontakt sehenden Bereich durch eine Ummantelung 51 ummantelt ist. Die Ummantelung 51 hat den Effekt, dass Füllmedium daran zu hindern in die Litzen des Drahtseils einzuströmen, wodurch ein Anstieg des fluidmechanischen Widerstands (z. B. auftretende Turbulenzen) durch die Bewegung des Drahtseils im Füllmedium verhindert werden kann. Dadurch kann die Gleiteigenschaft des Kraftübertragungskörper 50 in dem Fluidmedium verbessert werden.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Vorrichtung

- 20: Aufnahme
- 21: Erstes Ende
- 22: Zweites Ende
- 23: Erste Kammer
- 24: Zweite Kammer
- 25: Innenraum der Aufnahme
- 26: Verschluss
- 27: Endanschlag
- 28: Dichteinsatz
- 29: Dichtkörper

- 30: Füllmedium
- 31: Spalt
- 33: Dichtlippe
- 35: Wirkkörper
- 37: Begrenzungselement
- 39: Manschette

- 40: Erster Wirkkörper
- 42: Rückstellelement
- 43: Erster Abschnitt des ersten Wirkkörpers
- 44: Zweiter Abschnitt des zweiten Wirkkörpers

- 50: Kraftübertragungskörper
- 51: Ummantelung
- 52: Öffnung
- 53: O-Ring

- 60: Zweiter Wirkkörper
- 61: Aufnahmeraum der zweite Wirkkörpers
- 64: Durchlassöffnung
- 70: Erstes Anbindungselement
- 72: Kopplungselement

- 80: Zweites Anbindungselement

- S: Strömungsrichtung
- B: Auszugsrichtung

## Patentansprüche

1. Vorrichtung (1) zur Stabilisierung von Bewegungen zweier sich relativ zueinander bewegbarer Teile eines Körperbereichs und/oder eines Sportgeräts, umfassend:
eine an einem ersten Teil eines Körperbereichs und/oder eines Sportgeräts fixierbare Aufnahme (20), wobei die Aufnahme (20) mit einem Füllmedium (30) gefüllt ist, und
mindestens eine Wirkköperanordnung mit einem Wirkkörper (35), der in der Aufnahme (20) verschiebbar aufgenommen ist und mit dem Füllmedium (30) interagieren kann,
einen an einem zweiten Teil desselben Körperbereichs und/oder desselben Sportgeräts fixierbaren Kraftübertragungskörper (50) zum Übertragen einer äußeren Kraft auf den Wirkkörper (35),
wobei der Wirkkörper (35) mindestens eine Durchlassöffnung (64) umfasst, durch welche das Füllmedium (30) strömen kann,
wobei die Wirkkörperanordnung eine Dichtlippe (33) zum Abdichten eines Spalts (31) zwischen einer Innenseite der Aufnahme (20) und einem seitlichen Bereich einer Außenseite der Wirkkörperanordnung (33, 35) aufweist, wobei die Dichtlippe (33) an einer Außenseite des Wirkkörpers (35) angeordnet ist
der Wirkkörper (35) einen ersten Wirkkörper (40) und einen zweiten Wirkkörper (60) umfasst, wobei der erste Wirkkörper (40) und der zweite Wirkkörper (60) in der Aufnahme (20) verschiebbar angeordnet sind und mit dem Füllmedium (30) interagieren können;
wobei der Kraftübertragungskörper (50) die äußere Kraft auf den ersten Wirkkörper (40) überträgt;
wobei der zweite Wirkkörper (60) über ein Kopplungselement (72) elastisch mit dem ersten Wirkkörper (40) gekoppelt ist,
wobei der zweite Wirkkörper (60) und/oder der erste Wirkkörper (40) mindestens eine Durchlassöffnung (64) aufweisen, durch welche das Füllmedium (30) strömen kann,
wobei der erste Wirkkörper (40) einen Ventilkörper bildet und der zweite Wirkkörper (60) einen Ventilsitz bildet, wobei die Dichtlippe (33) an dem zweiten Wirkkörper (60) angeordnet ist,
**dadurch gekennzeichnet, dass**
die Wirkkörperanordnung einen an der Innenseite der Aufnahme (20) anliegenden Bereich der Dichtlippe aufweist, der in einem Winkel (α, β) zu einem seitlichen Bereich der Außenseite des Wirkkörpers (35) verläuft, wobei der Winkel (α, β) veränderlich ist, um Größenschwankungen des Spalts (31) zwischen der Innenseite der Aufnahme (20) und dem seitlichen Bereich der Außenseite der Wirkkörperanordnung ausgleichen zu können und den Spalt (31) auch bei derartigen Größenschwankungen abzudichten.

2. Vorrichtung (1) nach Anspruch 1 wobei der veränderliche Winkel (α, β) eine Größenschwankung des Spalts (31) von bis zu 15%, bezogen auf die ursprüngliche Größe des Spalts ausgleichen kann.

3. Vorrichtung (1) nach einem der vorhergehenden Ansprüche, wobei das Füllmedium (30) ab einer um mehr als 15% vergrößerten Größe des Spalts (31), bezogen auf die ursprüngliche Größe des Spalts (31), durch den Spalt (31) strömbar ist, um einen kritischen Innendruck in der Aufnahme (20) zu vermeiden.

4. Vorrichtung (1) nach einem der vorhergehenden Ansprüche, wobei die Dichtlippe (33) und der Wirkkörper (35) einstückig ausgebildet sind.

5. Vorrichtung (1) nach einem der vorhergehenden Ansprüche, wobei die Aufnahme (20) eine Öffnung (52) umfasst, wobei der Kraftübertragungskörper (50) durch die Öffnung (52) verläuft, und wobei die Dichtlippe (33) an einem der Öffnung (52) der Aufnahme (20) zugewandten Ende des Wirkkörpers (35) angeordnet ist.

6. Vorrichtung (1) nach einem der vorhergehenden Ansprüche, wobei sich die Dichtlippe (33) zumindest teilweise in Richtung der Öffnung (52) der Aufnahme (20) über den Wirkkörper (35) hinaus erstreckt, um einen Vorsprung in eine Auszugsrichtung des Wirkkörpers zu bilden.

7. Vorrichtung (1) nach einem der vorhergehenden Ansprüche, wobei die Dichtlippe (33) zum Schließen des Spalts (31) zwischen der Wirkkörperanordnung und der Aufnahme (20) schirmartig von dem Wirkkörper (35) abspreizbar ist.

8. Vorrichtung (1) nach einem der vorhergehenden Ansprüche, wobei die Dichtlippe (33) ein Elastizitätsmodul im Bereich von 300 bis 700 MPa aufweist.

9. Vorrichtung (1) nach einem der vorhergehenden Ansprüche, wobei die Dichtlippe (33) ab einem bestimmten Schwellwert einer auf den Kraftübertragungskörper wirkenden Kraft und einem daraus resultierenden bestimmten Druck in der Aufnahme, bevorzugt in einer ersten Kammer (24) in der Aufnahme (20), ein Strömen von Füllmedium durch den Spalt (31) zulässt, um einen kritischen Innendruck in der Aufnahme zu vermeiden.

10. Vorrichtung (1) nach einem der vorhergehenden Ansprüche, wobei um eine Öffnung (52) der Aufnahme (20), ein Endanschlag (21) an der Aufnahme (20) angeordnet ist, um ein direktes Aufprallen des Wirkkörpers (35) auf die Aufnahme (20) zu vermeiden.

11. Vorrichtung (1) gemäß einem der vorhergehenden Ansprüche, wobei die Dichtlippe (31) durch ein Zweikomponenten-Spritzgussherstellungsverfahren an den Wirkköper (35, 60) angespritzt ist, wobei zumindest die Dichtlippe (33) ein Polymer aufweist.

## Claims

1. Device (1) for stabilizing movements of two parts of a body region and/or a sports device that can move relative to each other, comprising:
a receptacle (20) that can be fixed to a first part of a body region and/or a sports device, wherein the receptacle (20) is filled with a filling medium (30), and
at least one active body arrangement with an active body (35) which is received displaceably in the receptacle (20) and can interact with the filling medium (30),
a force transmission body (50) which can be fixed to a second part of the same body region and/or the same sports device for transmitting an external force to the active body (35),
wherein the active body (35) comprises at least one passage opening (64) through which the filling medium (30) can flow,
wherein the active body arrangement has a sealing lip (33) for sealing a gap (31) between an inner side of the receptacle (20) and a lateral region of an outer side of the active body arrangement (33, 35), wherein the sealing lip (33) is arranged on an outer side of the active body (35),
the active body (35) comprises a first active body (40) and a second active body (60), wherein the first active body (40) and the second active body (60) are arranged displaceably in the receptacle (20) and can interact with the filling medium (30);
wherein the force transmission body (50) transmits the external force to the first active body (40);
wherein the second active body (60) is elastically coupled to the first active body (40) via a coupling element (72),
wherein the second active body (60) and/or the first active body (40) have at least one passage opening (64) through which the filling medium (30) can flow,
wherein the first active body (40) forms a valve body and the second active body (60) forms a valve seat, wherein the sealing lip (33) is arranged on the second active body (60),
**characterized in that**
the active body arrangement has a region of the sealing lip bearing on the inner side of the receptacle (20), which region extends at an angle (α, β) to a lateral region of the outer side of the active body (35), wherein the angle (α, β) can be varied in order to compensate for size fluctuations of the gap (31) between the inner side of the receptacle (20) and the lateral region of the outer side of the active body arrangement and to seal the gap (31) even in the event of such size fluctuations.

2. Device (1) according to claim 1, wherein the variable angle (α, β) can compensate for a size variation of the gap (31) of up to 15%, relative to the original size of the gap.

3. Device (1) according to any one of the preceding claims, wherein the filling medium (30) can flow through the gap (31) when the size of the gap (31) increases by more than 15% relative to the original size of the gap (31), in order to avoid a critical internal pressure in the receptacle (20).

4. Device (1) according to any one of the preceding claims, wherein the sealing lip (33) and the active body (35) are formed in one piece.

5. Device (1) according to any one of the preceding claims, wherein the receptacle (20) comprises an opening (52), wherein the force transmission body (50) extends through the opening (52), and wherein the sealing lip (33) is arranged at an end of the active body (35) facing the opening (52) of the receptacle (20).

6. Device (1) according to any one of the preceding claims, wherein the sealing lip (33) extends at least partially in the direction of the opening (52) of the receptacle (20) beyond the active body (35) to form a projection in an extraction direction of the active body.

7. Device (1) according to any one of the preceding claims, wherein the sealing lip (33) can spread away from the active body (35) in a shield-like manner to close the gap (31) between the active body arrangement and the receptacle (20).

8. Device (1) according to any one of the preceding claims, wherein the sealing lip (33) has a Young's modulus in the range of 300 to 700 MPa.

9. Device (1) according to any one of the preceding claims, wherein the sealing lip (33) allows a filling medium to flow through the gap (31) from a certain threshold value of a force acting on the force transmission body and a resulting certain pressure in the receptacle, preferably in a first chamber (24) in the receptacle (20), in order to prevent a critical internal pressure in the receptacle.

10. Device (1) according to any one of the preceding claims, wherein an end stop (21) is arranged on the receptacle (20), around an opening (52) of the receptacle (20), in order to prevent the active body (35) from impacting directly on the receptacle (20).

11. Device (1) according to any one of the preceding claims, wherein the sealing lip (31) is injection molded onto the active body (35, 60) by a two-component injection molding method, wherein at least the sealing lip (33) has a polymer.

## Revendications

1. Dispositif (1) de stabilisation de mouvements de deux parties mobiles l'une par rapport à l'autre d'une zone corporelle et/ou d'un appareil de sport comprenant :
un logement (20) pouvant être fixé au niveau d'une première partie d'une zone corporelle et/ou d'un appareil de sport, dans lequel le logement (20) est rempli d'un milieu de remplissage (30) et
au moins un ensemble de corps actif avec un corps actif (35) qui est logé de manière déplaçable dans le logement (20) et peut interagir avec le milieu de remplissage (30),
un corps de transmission de force (50) pouvant être fixé au niveau d'une seconde partie de la même zone de corps et/ou du même appareil de sport pour la transmission d'une force extérieure au corps actif (35),
dans lequel le corps actif (35) comprend au moins une ouverture de passage (64) à travers laquelle le milieu de remplissage (30) peut s'écouler,
dans lequel l'ensemble de corps actif présente une lèvre d'étanchéité (33) pour rendre étanche une fente (31) entre un côté intérieur du logement (20) et une zone latérale d'un côté extérieur de l'ensemble de corps actif (33, 35), dans lequel la lèvre d'étanchéité (33) est agencée au niveau d'un côté extérieur du corps actif (35)
le corps actif (35) comprend un premier corps actif (40) et un second corps actif (60), dans lequel le premier corps actif (40) et le second corps actif (60) sont agencés de manière déplaçable dans le logement (20) et peuvent interagir avec le milieu de remplissage (30) ;
dans lequel le corps de transmission de force (50) transmet la force extérieure au premier corps actif (40) ;
dans lequel le second corps actif (60) est couplé élastiquement au premier corps actif (40) par le biais d'un élément de couplage (72),
dans lequel le second corps actif (60) et/ou le premier corps actif (40) présentent au moins une ouverture de passage (64) à travers laquelle le milieu de remplissage (30) peut s'écouler,
dans lequel le premier corps actif (40) forme un corps de soupape et le second corps actif (60) forme un siège de soupape, dans lequel la lèvre d'étanchéité (33) est agencée au niveau du second corps actif (60),
**caractérisé en ce que**
l'ensemble de corps actif présente une zone reposant contre le côté intérieur du logement (20) de la lèvre d'étanchéité, zone qui s'étend selon un angle (α, β) jusqu'à une zone latérale du côté extérieur du corps actif (35), dans lequel l'angle (α, β) est modifiable afin de pouvoir compenser des oscillations de grandeur de la fente (31) entre le côté intérieur du logement (20) et la zone latérale du côté extérieur de l'ensemble de corps actif et de rendre étanche la fente (31) également en cas de telles oscillations de grandeur.

2. Dispositif (1) selon la revendication 1, dans lequel l'angle modifiable (α, β) peut compenser une oscillation de grandeur de la fente (31) jusqu'à 15 % maximum par rapport à la grandeur d'origine de la fente.

3. Dispositif (1) selon l'une quelconque des revendications précédentes, dans lequel le milieu de remplissage (30) peut s'écouler à partir d'une grandeur de la fente (31) augmentée de plus de 15%, par rapport à la grandeur d'origine de la fente (31), à travers la fente (31) afin d'éviter une pression interne critique dans le logement (20).

4. Dispositif (1) selon l'une quelconque des revendications précédentes, dans lequel la lèvre d'étanchéité (33) et le corps actif (35) sont réalisés d'un seul tenant.

5. Dispositif (1) selon l'une quelconque des revendications précédentes, dans lequel le logement (20) comprend une ouverture (52), dans lequel le corps de transmission de force (50) s'étend à travers l'ouverture (52), et dans lequel la lèvre d'étanchéité (33) est agencée au niveau d'une extrémité du corps actif (35) tournée vers l'ouverture (52) du logement (20).

6. Dispositif (1) selon l'une quelconque des revendications précédentes, dans lequel la lèvre d'étanchéité (33) s'étend au moins partiellement en direction de l'ouverture (52) du logement (20) au-delà du corps actif (35) afin de former une saillie dans une direction d'extraction du corps actif.

7. Dispositif (1) selon l'une quelconque des revendications précédentes, dans lequel la lèvre d'étanchéité (33) peut être écartée du corps actif (35) à la manière d'un parapluie pour la fermeture de la fente (31) entre l'ensemble de corps actif et le logement (20).

8. Dispositif (1) selon l'une quelconque des revendications précédentes, dans lequel la lèvre d'étanchéité (33) présente un module de Young dans la plage de 300 à 700 MPa.

9. Dispositif (1) selon l'une quelconque des revendications précédentes, dans lequel la lèvre d'étanchéité (33) autorise un écoulement de milieu de remplissage à travers la fente (31) afin d'éviter une pression intérieure critique dans le logement à partir d'une valeur seuil déterminée d'une force agissant sur le corps de transmission de force et d'une pression déterminée en résultant dans le logement, de préférence dans une première chambre (24) dans le logement (20).

10. Dispositif (1) selon l'une quelconque des revendications précédentes, dans lequel une butée d'extrémité (21) est agencée au niveau du logement (20) autour d'une ouverture (52) du logement (20) afin d'éviter un impact direct du corps actif (35) sur le logement (20).

11. Dispositif (1) selon l'une quelconque des revendications précédentes, dans lequel la lèvre d'étanchéité (31) est moulée par injection sur le corps actif (35, 60) par un procédé de fabrication par moulage par injection à deux composants, dans lequel au moins la lèvre d'étanchéité (33) présente un polymère.
